(19) Europäisches Patentamt / European Patent Office / Office européen des brevets

(11) **EP 3 801 420 B1**

(12) **EUROPEAN PATENT SPECIFICATION**

(45) Date of publication and mention of the grant of the patent:
**06.03.2024 Bulletin 2024/10**

(21) Application number: **18731011.5**

(22) Date of filing: **05.06.2018**

(51) International Patent Classification (IPC):
*A61F 13/49* (2006.01)     *A61F 13/496* (2006.01)
*A61F 13/513* (2006.01)     *A61F 13/514* (2006.01)

(52) Cooperative Patent Classification (CPC):
**A61F 13/496; A61F 13/49006; A61F 13/51394; A61F 13/51401; A61F 13/51496**

(86) International application number:
**PCT/EP2018/064788**

(87) International publication number:
**WO 2019/233569 (12.12.2019 Gazette 2019/50)**

(54) **ARRAY OF DISPOSABLE PANT ARTICLES AND METHOD FOR PRODUCING AN ARRAY OF DISPOSABLE PANT ARTICLES**

ANORDNUNG VON WEGWERFHÖSCHENARTIKELN UND VERFAHREN ZUR HERSTELLUNG EINER ANORDNUNG VON WEGWERFHÖSCHENARTIKELN

RÉSEAU D'ARTICLES DE CULOTTE JETABLE ET PROCÉDÉ DE PRODUCTION D'UN RÉSEAU D'ARTICLES DE CULOTTE JETABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**14.04.2021 Bulletin 2021/15**

(73) Proprietor: **Essity Hygiene and Health Aktiebolag**
**405 03 Göteborg (SE)**

(72) Inventors:
• **LJUNGBERG, Karin**
  **405 03 Göteborg (SE)**
• **ERIKSSON, Katarina**
  **405 03 Göteborg (SE)**
• **BÄCK, Lucas**
  **405 03 Göteborg (SE)**
• **STENBERG, Anna**
  **405 03 Göteborg (SE)**

(74) Representative: **Nederlandsch Octrooibureau**
**P.O. Box 29720**
**2502 LS The Hague (NL)**

(56) References cited:
EP-A1- 1 704 842      WO-A1-2007/024327
WO-A1-2010/050853     WO-A1-2010/050854
WO-A1-2016/182484     WO-A1-2018/097770
WO-A2-2006/015207     US-A1- 2012 323 204

## Description

### Field of the disclosure

The present disclosure relates to an array of disposable pant articles. It also relates to a method for producing an array of disposable pant articles.

### Background art

Disposable absorbent articles, for example in the form of incontinence liners, baby diapers and sanitary napkins, are well known. The general purpose of such absorbent articles is to absorb, distribute and store various types of body exudates, while providing a high level of comfort and sense of dryness to the wearer during use of the absorbent article. Also, such an absorbent article is configured to prevent the wearer from getting the clothes soiled by such body exudates.

Absorbent articles in the form of incontinence articles are used to protect a wearer against urine leakage. An incontinence article can be configured for example as a pant diaper, a sanitary pant or an incontinence pant adapted for use by a baby, child or adult, male or female user. Also, an incontinence article is designed with an absorption capacity which is adapted to absorb the fluid that is expected to be released into the article when it is worn. Incontinence articles are used to assist persons with incontinence so that they can maintain a normal lifestyle without any inconvenience caused by incontinence.

Prior art incontinence absorbent products are usually white, with or without print. There are also coloured products on the market which are coloured on the outside. However, many prior art products remain very diaper-like and are therefore often disliked by users, who want a discreet product. They do not want to be continuously reminded that they are using an incontinence product and they do not want to show other people that they are using one, while at the same time they want to feel secure when using the products.

Coloured materials sometimes increase manufacturing cost. Coloured materials are more expensive per se because the supplier wastes volumes of the material due to the cleaning needed after production of a batch. Furthermore, the use of coloured materials in producing absorbent articles is also avoided due to risk of colour contamination of white layers of the product or subsequent products, which may likewise lead to volumes of articles being scrapped and the need for cleaning after production of a batch of articles.

WO 2010/050854 A1 and WO 2010/050853 A1 disclose disposable pant article comprising an information tag arranged on the inside of the waist portion. No specific colour requirements are defined. WO 2016/182484 A1 and EP 1 704 842 A1 disclose pant articles having colour requirements for the outside surface of the pant article and do not disclose a tag. WO 2007/024327 A1 and US 2012/323204 A1 disclose pant articles having colour requirements but not for the topsheet covering the absorbent core. Also, no tag is disclosed. WO 2006/015207 A2 discloses a disposable absorbent article with colour requirements for elements disposed on regions of that article and does not disclose a tag. WO 2018/097770 A1 discloses an array of disposable pant articles.

There is thus a need for more discreet disposable pant products which can be manufactured at lower cost.

### Summary of the disclosure

It is a first aim of the present disclosure to provide an array of disposable pant products which can be manufactured at lower cost.

It is a second aim of the present disclosure to provide an array of more discreet disposable pant products.

It is a third aim of the present disclosure to provide an array of more underwear like disposable pant products.

It is a fourth aim of the present disclosure to provide a lower cost manufacturing method for an array of disposable pant products.

The disclosure provides, according to a first aspect, an array comprising at least one first disposable pant article and at least one second disposable pant article, wherein each first disposable pant article has a first structure, comprising a waist portion which in use surrounds a user's waist and a crotch portion connecting front and back portions of the waist portion, the crotch portion comprising an absorbent core for absorbing body exudates and a liquid-permeable topsheet covering the absorbent core, wherein the article has an inside surface which in use faces the user's skin and an outside surface which is opposite the inside surface and in use faces the user's clothing; wherein the article is composed of a plurality of fabrics which define the colours of the inside and outside surfaces of the article, the liquid-permeable topsheet being one of the fabrics; wherein an absorption zone is defined as the part of the inside surface, on the topsheet, under which the absorbent core is located, and wherein a surrounding zone is defined as the part of the inside surface, on the topsheet, adjacent to and at least partly surrounding the absorption zone; and each second disposable pant article has a second structure, comprising a waist portion which in use surrounds a user's waist and a crotch portion connecting front and back portions of the waist portion, the crotch portion comprising an absorbent core for absorbing body exudates and a liquid-permeable topsheet covering the absorbent core while indicating an absorption zone where the absorbent core is located, wherein the article has an inside surface which in use faces the user's skin and an outside surface which is opposite the inside surface and in use faces the user's clothing; wherein the article is composed of a plurality of fabrics which define the colours of the inside and outside surfaces of the article, the liquid-permeable topsheet being one of the fabrics; wherein an absorption zone is defined as the part of the inside surface, on the topsheet, under which the absorbent core is located, and wherein a surrounding zone is defined as the part of the inside surface, on the topsheet, adjacent to and at least partly surrounding the absorption zone; wherein for each first disposable pant article, at least the outside surface and said surrounding zone have substantially the same first colour with, in the CIE L*a*b* colour space, L* values less than 80 and mutual colour differences $\Delta E*ab$ less than 10; for each second disposable pant article, at least the outside surface and said surrounding zone have substantially the same second colour with L* values less than 80 and mutual colour differences $\Delta E*ab$ less than 10; said outside surface of said first and/or second disposable pant article comprises outside surface portions, together forming said outside surface, and said inside surface of said first and/or second disposable pant article comprises inside surface portions, together forming said inside surface, and all the outside and inside surface portions of said first article, possibly except in said absorption zone, have L* values less than 80 and mutual colour differences $\Delta E*ab$ less than 10 and/or all the outside and inside surface portions of said second article, possibly except in said absorption zone, have L* values less than 80 and mutual colour differences $\Delta E*ab$ less than 10; each of the first and second disposable pant articles comprises an information tag arranged on the inside of said waist portion and providing a visual indicator for differentiating between the front and back portions of the respective article, the material of the information tag being in a third colour which is different from the first and second colours by a colour difference $\Delta E*ab$ more than 20; and the first structure is structurally different from the second structure and/or the first colour is different from the second colour by a colour difference $\Delta E*ab$ more than 10.

As used herein, "Array" means a display of packages or products comprising disposable articles of different sizes, having like article constructions (same absorbent inserts, chassis constructions, material etc. [compositionally and/or structurally] or disposable articles of different types within a product range/assortment, for example if the product range/assortment is a pant absorbent product the different types can be a laminate pant or a threaded pant. The packages having the same brand and/or sub- brand, and said packages oriented in proximity to each other in a given area of a retail store or products arranged together as product offer in a catalogue.

An "Array" being a display of packages or products comprising disposable articles of e.g. different sizes, having like article constructions (same absorbent inserts, chassis constructions, material etc [compositionally and/or structurally] is marketed as a line-up of products normally having like packaging elements (e.g., packaging material type, film, paper, dominant color, design theme, etc.) that convey to consumers that the different individual packages are part of a larger line-up and the products within the array are e.g. designed for different sizes. Such arrays often have the same brand, and same sub-brand. Such arrays also often have the same trademarks, including trademarks of the brand, sub- brand, and/or features and/or benefits across the line-up.

In the array according to the present disclosure, all articles have the same, or substantially the same, information tag in substantially the same location on the waist portion. Further, the colour is different from the colour of the article. So the information tags are distinguishable and located to indicate the inside of the article to the user. The disposable pant article is a disposable absorbent article.

In embodiments of the array according to the present disclosure, the articles may be "fully coloured", i.e. the outer and inner materials/fabrics used for the waist and crotch portions may all have substantially the same colour. As a result, the problem of colour contamination during manufacturing may be avoided. Other materials which are arranged under the outer and inner fabrics may also be coloured and influence the colour of the absorbent article.

In embodiments of the array according to the present disclosure, said outside surface of said first and/or second disposable pant article comprise outside surface portions, together forming said outside surface, and said inside surface of said first and/or second disposable pant article may comprise inside surface portions, together forming said inside surface, and all the inside and outside surface portions of said first article except in said absorption zone, have L* values less than 80 and mutual colour differences $\Delta E*ab$ less than 10 and/or all the inside and outside surface portions of said second article except in said absorption zone, have L* values less than 80 and mutual colour differences $\Delta E*ab$ less than 10.

In embodiments of the array according to the present disclosure, said outside surface of said first and/or second disposable pant article may comprise outside surface portions, together forming said outside surface, and said inside surface of said first and/or second disposable pant article may comprises inside surface portions, together forming said inside surface, and all the inside and outside surface portions of said first article except in said absorption zone, have L* values less than 60 and mutual colour differences $\Delta E*ab$ less than 10 and/or all the inside and outside surface portions of said second article except in said absorption zone, have L* values less than 60 and mutual colour differences $\Delta E*ab$ less than 10.

In embodiments of the array according to the present disclosure, said outside surface of said first and/or second disposable pant article may comprise outside surface portions, together forming said outside surfaces, and said inside surface of said first and/or second disposable pant article may comprises inside surface portions, together forming said inside surface, and all the inside and outside surface portions of said first article, have L* values less than 80 and mutual colour differences $\Delta E^*ab$ less than 10 and/or all the inside and outside surface portions of the second disposable pant article, have L* values less than 80 and mutual colour differences $\Delta E^*ab$ less than 10.

In embodiments of the array according to the present disclosure, said outside surface of said first and/or second disposable pant article may comprise outside surface portions, together forming said outside surfaces, and said inside surface of said first and/or second disposable pant article may comprise inside surface portions, together forming said inside surface, and all the inside and outside surface portions of said first article, have L* values less than 60 and mutual colour differences $\Delta E^*ab$ less than 10 and/or all the inside and outside surface portions of the second disposable pant article, have L* values less than 60 and mutual colour differences $\Delta E^*ab$ less than 10.

One of the outer portions of the outer surface may be a liquid-impermeable backsheet of the crotch portion. One of the portions of the outer surfaces may be an elastic non-woven laminate forming the waist portion or one of the sheets forming the elastic non-woven laminate. Such an elastic laminate may comprise two non-elastic non-woven materials with elastic means, such as an elastic film and/or elastic strands, sandwiched between the two non-woven materials. Alternatively, an elastic non-woven can form the waist portion. Simultaneously, the elastic laminate or the elastic nonwoven forming one portion of the outer surface of the waist portion may be a portion of the inside surface of the waist portion. The topsheet with underlying material may also form a portion of the inside surface.

Due to the colours of the outside surface and the inside surface at least in the area of the surrounding zone being substantially the same, i.e. a $\Delta E^*ab$ less than 10, the pant article of the present disclosure is more uniform in colour As a result of being more uniform in colour, the pant articles according to the present disclosure can meet a demand for e.g. incontinence articles which have a design such that they resemble regular underwear, while still giving the wearer a sense of security (by indicating presence of the absorbent core). The discreet pant article can give the wearer a higher level of self-confidence, comfort and self-esteem and can provide incontinence protection for users having different lifestyles.

In embodiments of the array according to the present disclosure, the absorption zone of each of said first and/or second disposable pant article may be, in the CIE L*a*b* colour space, an L* value less than 90 and a colour difference $\Delta E^*ab$ more than 5 with respect to said surrounding zone and/or said outer surface.

In embodiments according to the disclosure, the structural difference may be that the first article is optimized for male users whereas the second article is optimized for female users. For example, the cut or shape of the first article may be different with respect to the second article, and/or the location and/or number of elastic threads may be different.

In embodiments according to the disclosure, the structural difference may be that the first and second articles have different sizes. Arrays according to the present disclosure may comprise articles of for example sizes XS (extra-small), S (small), M (medium), L (large), XL (extra-large), or others.

In embodiments according to the disclosure, the structural difference may be that the first article is a threaded pant article comprising elastic threads in the waist portion, whereas the second article is not such a threaded pant article.

In embodiments according to the disclosure, the structural difference may be that the first article is a layered pant article comprising an elastic film laminate in the waist portion, whereas the second article is not such a layered pant article.

In embodiments according to the disclosure, the first and second colours may be substantially the same. The first and second colours may be substantially the same, i.e. $\Delta E^*ab$ less than 10 Arrays according to the disclosure may only comprise articles of substantially the same colour with structural differences.

In embodiments according to the disclosure, the first and second colours may be different by a colour difference $\Delta E^*ab$ more than 10, i.e. arrays according to the disclosure may comprise articles of different colours, with and without structural differences.

In embodiments according to the disclosure, arrays of absorbent pant articles may comprise any combination of male and female pant articles, articles of different sizes, threaded-type and layered-type pant articles and/or articles of different colours.

According to the disclosure, the first and second colours have L* values less than 80.

In embodiments according to the disclosure, the first and second colours have L* values less than 60.

In embodiments according to the disclosure, for each of said first and second articles, the information tag is arranged on the back portion of the waist portion.

In embodiments according to the disclosure, for each of said first and second articles, the absorbent core may be visible through the liquid-permeable topsheet. For example, the presence of the absorbent core (i.e. the absorption zone) may be indicated merely by visibility of the absorbent core, through the topsheet. The core may be in a material of lighter colour than the topsheet

In embodiments according to the disclosure, the information tags may be printed or embossed, i.e. information may be provided on the tags by print and/or embossing. Example of such information are lot codes, production codes, logos and/or size indications.

In a second aspect, which may be combined with the other aspects and embodiments described herein, the disclosure provides a method for producing an array of disposable pant articles, comprising the steps of: producing a plurality of first articles having a first structure, comprising a waist portion which in use surrounds a user's waist and a crotch portion connecting front and back portions of the waist portion, the crotch portion comprising an absorbent core for absorbing body exudates and a liquid-permeable topsheet covering the absorbent core wherein the article has an inside surface which in use faces the user's skin and an outside surface which is opposite the inside surface and in use faces the user's clothing; wherein the article is composed of a plurality of fabrics which define the colours of the inside and outside surfaces of the article, the liquid-permeable topsheet being one of the fabrics; wherein an absorption zone is defined as the part of the inside surface, on the topsheet, under which the absorbent core is located, and wherein a surrounding zone is defined as the part of the inside surface, on the topsheet, adjacent to and at least partly surrounding the absorption zone; wherein for each first disposable pant article, at least the outside surface and said surrounding zone have substantially the same first colour with, in the CIE L*a*b* colour space, L* values less than 80 and mutual colour differences $\Delta E*ab$ less than 10; producing a plurality of second articles having a first structure, comprising a waist portion which in use surrounds a user's waist and a crotch portion connecting front and back portions of the waist portion, the crotch portion comprising an absorbent core for absorbing body exudates and a liquid-permeable topsheet covering the absorbent core wherein the article has an inside surface which in use faces the user's skin and an outside surface which is opposite the inside surface and in use faces the user's clothing; wherein the article is composed of a plurality of fabrics which define the colours of the inside and outside surfaces of the article, the liquid-permeable topsheet being one of the fabrics; wherein an absorption zone is defined as the part of the inside surface, on the topsheet, under which the absorbent core is located, and wherein a surrounding zone is defined as the part of the inside surface, on the topsheet, adjacent to and at least partly surrounding the absorption zone; wherein for each second disposable pant article, at least the outside surface and said surrounding zone have substantially the same second colour with L* values less than 80 and mutual colour differences $\Delta E*ab$ less than 10; wherein said outside surface of said first and/or second disposable pant article comprises outside surface portions, together forming said outside surface, and said inside surface of said first and/or second disposable pant article comprises inside surface portions, together forming said inside surface, and all the outside and inside surface portions of said first article, possibly except in said absorption zone, have L* values less than 80 and mutual colour differences $\Delta E*ab$ less than 10 and/or all the outside and inside surface portions of said second article, possibly except in said absorption zone, have L* values less than 80 and mutual colour differences $\Delta E*ab$ less than 10; wherein for each of said first and second disposable pant articles the first structure is structurally different from the second structure and/or the first colour is different from the second colour by a colour difference $\Delta E*ab$ more than 10; and attaching information tags on the inside of said waist portions of each of the first and second articles, thereby providing a visual indicator for differentiating between the front and back portions of the respective article; the material of the information tag being in a third colour which is different from the first and second colours by a colour difference $\Delta E*ab$ more than 20.

For all of the first and second articles, the first structure is structurally different from the second structure and/or the first colour is different from the second colour by a colour difference $\Delta E*ab$ more than 10.

At least the outside surface and the surrounding zone, i.e. the zone on the liquid-permeable topsheet at least partly surrounding the absorption zone of at least one of said first and second articles have substantially the same colour with, in the CIE L*a*b* colour space, mutual colour differences $\Delta E*ab$ less than 10.

At least the outside surface and the surrounding zone, i.e. the zone on the liquid-permeable topsheet at least partly surrounding the absorption zone of both said first and second articles may have substantially the same colour with, in the CIE L*a*b* colour space, mutual colour differences $\Delta E*ab$ less than 10.By using fabrics of the same, or substantially the same colour, the need for cleaning between production runs of first articles and second articles to avoid colour contamination can be avoided, resulting in a more efficient and thus more economical manufacturing process.

**Brief description of the drawings**

The present disclosure will be discussed in more detail below, with reference to the attached drawings.

Figs. 1-7 show a first set of embodiments of articles of an array according to the present disclosure.
Fig. 1 is a perspective view of an embodiment of an absorbent pant article.
Fig. 2 is a simplified plan view of the absorbent pant article in its flat, non-contracted state.
Fig. 3 is a cross section according to the line III-III in Fig. 2.
Fig. 4 is a cross section through an elastic laminate according to the invention.
Fig. 5 is a schematic cross section according to the line V-V in Fig. 2.
Fig. 6 is a schematic cross section similar to Fig. 5 but illustrating another embodiment.
Fig. 7 is a plan view similar to Fig. 2 but showing a further embodiment of an absorbent pant article.
Figs. 8-13 show a second set of embodiments of articles of an array according to the present disclosure.

Figure 8a shows a perspective front view of the absorbent article according to the disclosure;

Figure 8b is a perspective view of an enlarged section of a waist edge forming part of the absorbent article;

Figure 9 shows a top view of the absorbent article;

Figure10 shows a schematic illustration of a manufacturing process for an absorbent article according to the disclosure;

Figures 11a-11fshow cross-sectional views of alternative embodiments of the absorbent article according to the disclosure;

Figure 12 shows a cross-sectional view of a crotch section of said absorbent article;

Figure 13a is a perspective view of the absorbent article as seen from the rear, showing a first configuration, which is adapted for the male anatomy; and

Figure 13b is a perspective view of the absorbent article as seen from the rear, showing a second configuration, which is adapted for the female anatomy.

Figs. 14-16 schematically show an embodiment of an array according to the present disclosure.

## Description of embodiments

The present disclosure will be described with respect to particular embodiments and with reference to certain drawings but the disclosure is not limited thereto but only by the claims. The drawings described are only schematic and are non-limiting. In the drawings, the size of some of the elements may be exaggerated and not drawn on scale for illustrative purposes. The dimensions and the relative dimensions do not necessarily correspond to actual reductions to practice of the disclosure.

Furthermore, the terms first, second, third and the like in the description and in the claims, are used for distinguishing between similar elements and not necessarily for describing a sequential or chronological order. The terms are interchangeable under appropriate circumstances and the embodiments of the disclosure can operate in other sequences than described or illustrated herein.

Moreover, the terms top, bottom, over, under and the like in the description and the claims are used for descriptive purposes and not necessarily for describing relative positions. The terms so used are interchangeable under appropriate circumstances and the embodiments of the disclosure described herein can operate in other orientations than described or illustrated herein.

Furthermore, the various embodiments, although referred to as "preferred" are to be construed as exemplary manners in which the disclosure may be implemented rather than as limiting the scope of the disclosure.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising A and B" should not be limited to devices consisting only of components A and B, rather with respect to the present disclosure, the only enumerated components of the device are A and B, and further the claim should be interpreted as including equivalents of those components.

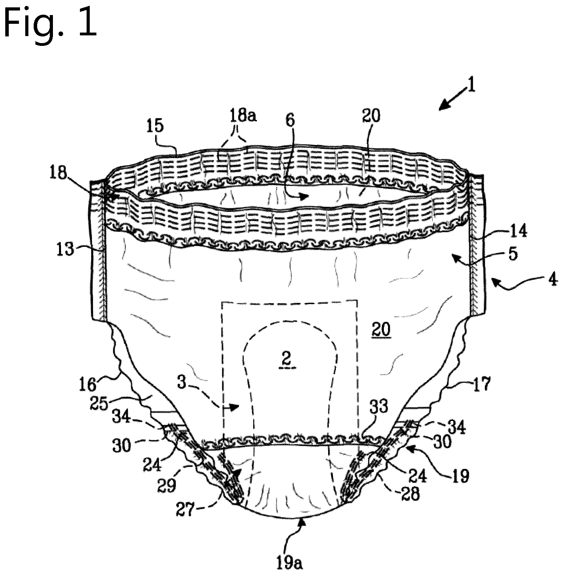

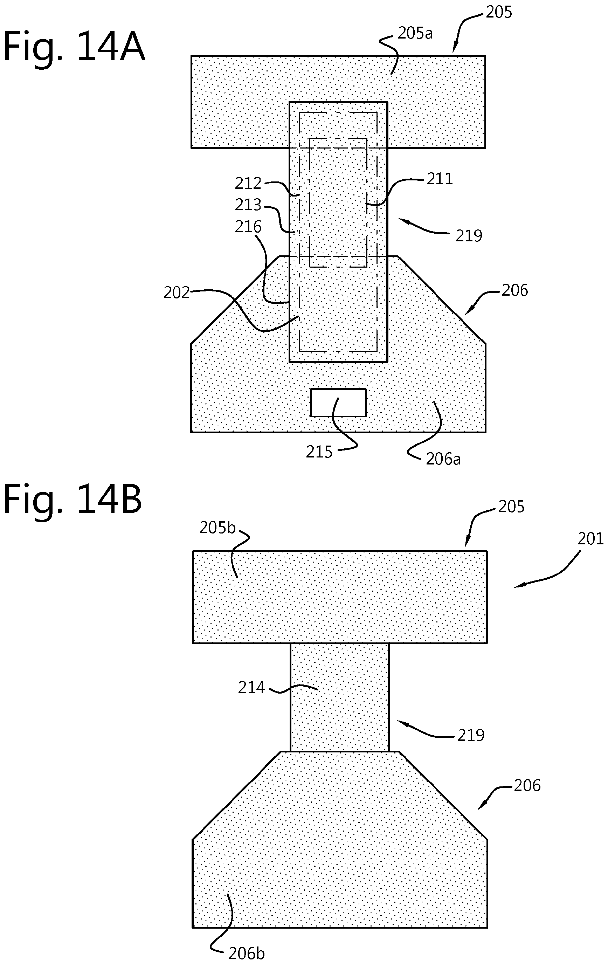

A first set of embodiments of absorbent articles according to the present disclosure will be described with reference to Figs. 1-7, in particular elastic film laminate-type pant articles. Fig. 14a and 14b schematically show the inside and outside of such articles.

Such elastic film laminate-type pant articles comprise waist and crotch portions which include a pant-shaped chassis and an absorbent core component integrated with the chassis. They are intended to fit comfortably and snugly about the wearer. It is further desirable that the articles are capable of being pulled up and down over the hips of the wearer to allow the wearer or caregiver to easily put on and remove the article when it has been soiled. Thereto, portions of the chassis are composed of an elastic laminate comprising first and second layers of fibrous material and an elastic film layer located there between. Such articles are for example known from WO 2005/122985, WO 2007/133127 and WO 2016/068764. Chassis composed of elastic laminates of this type are relatively smooth with less wrinkles as compared to laminates comprising elastic strands sandwiched between fibrous materials, as disclosed in for example 2003/0028166 and US 2009/0275911.

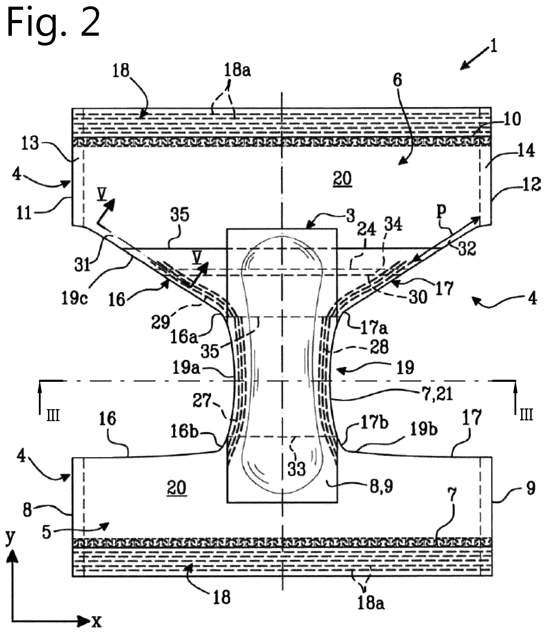

The elastic film laminate-type absorbent article 1 disclosed in Fig. 1 and 2 is intended to enclose the lower part of the wearer's trunk like a pair of underwear. The article 1 comprises a core region 3 with an absorbent core 2, which defines the "absorption zone". The article further comprises a chassis 4. The article has a longitudinal (y) and a transverse direction (x). The chassis 4 comprises a front portion 5 and a back portion 6, together forming a waist portion which in use surrounds the wearer's waist. The front portion 5 has a front transverse edge 7 and first and second longitudinal side edges 8 and 9. The back portion 6 has a back transverse end edge 10 and first and second longitudinal side edges 11 and 12. The front and back portions 5 and 6 are joined to each other along their respective first and second longitudinal side edges by ultrasonic welds, glue strings or the like to form first and second side seams 13 and 14 and to define a waist-opening 15.

The chassis may further comprise an elastic waist band 18 comprising elongated elastic members 18a. The elastic waist band 18 is secured to the transverse end edges 7 and 10 of the front and back portions 5 and 6. The elastic waist band portions 18 are joined to each other along said side seams 13 and 14. Alternatively, the elastic waist band may comprises first and second plies of substantially non-elastic nonwoven material that is elasticized by one or more elongate elastic members, such as elastic threads or bands. The first and second plies can be formed from a single layer of material that is folded over onto itself or can be made from two separate strips of material. A further option is to create the portion from one or more non-elastic nonwoven layers that are also parts of the front and back panels and form continuous extensions thereof. It is also conceivable to form an elastic waist feature by double-folding portions along the waist edges of the elastic front and back panels and optionally supplementing the folded portions by additional elastic elements.

The article 1 further comprises a connecting portion 19 located between the front portion 5 and the back portion 6 in the longitudinal direction of the article. The connecting portion 19 defines a crotch portion 19a and first and second leg openings 16 and 17. The boundary between the connecting portion 19 and the front and back portions 5 and 6 is along a transverse line extending between the lower edges of the side seams 13 and 14 adjacent the leg openings 16 and 17. The entire leg openings 16 and 17 are thus located in the connecting portion 19. The core region 3 is located in the connecting portion 19 and may extend into the front and/or back portions 5 and 6.

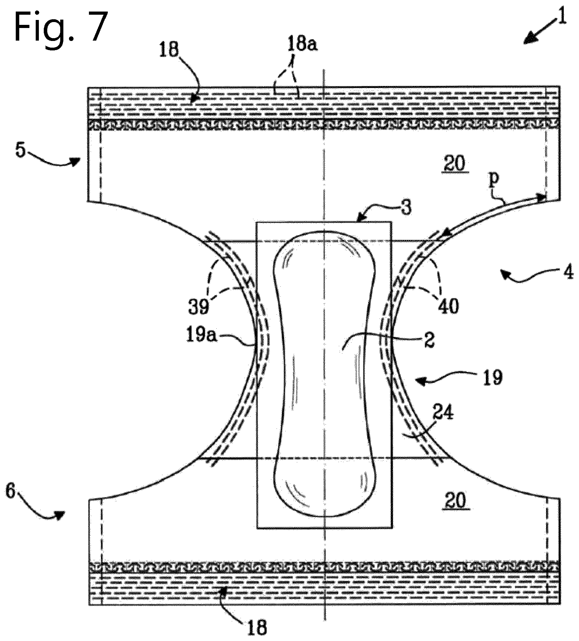

In the pant article shown in Fig. 2 the connecting portion 19 has defined points in the leg openings 16 and 17 where the width of the connecting portion 19 increases abruptly. These points are denoted 16a, 16b, 17a, 17b. In the pant article shown in Fig. 7 the leg openings 16 and 17 have a more or less continuous curvature with no such abrupt change of the radius of curvature.

A backsheet material 25 underlies the absorbent core 2 and adjacent areas immediately outside the absorbent core 2. The backsheet is preferably liquid-impervious. The area covered by the backsheet 25 is defined as the core region 3. A liquid-pervious topsheet material 26 is arranged on the wearer-facing side of the absorbent core 2, so that the absorbent core 2 is enclosed between the backsheet material 25 and the topsheet material 26. The absorbent core 2, the backsheet 25 and the topsheet 26 form an absorbent assembly.

The preferably liquid-impervious materials used for the backsheet 25 may be a thin plastic film, e.g. a polyethylene or polypropylene film, a nonwoven material coated with a liquid impervious material, a hydrophobic nonwoven material which resists liquid penetration or a laminate comprising plastic films and nonwoven materials. The backsheet 25 may be breathable so as to allow vapour to escape from the absorbent core, while still preventing liquids from passing therethrough. Examples of breathable backsheet materials are porous polymeric films, nonwoven laminates from spunbond and meltblown layers, laminates from porous polymeric films and nonwovens.

The liquid-pervious materials used for the topsheet 26 may be a nonwoven material, e.g. spunbond, meltblown, carded, hydroentangled, wetlaid etc. Suitable nonwoven materials can be composed of manmade fibres, such as polyester, polyethylene, polypropylene, viscose etc. or natural fibers, such as woodpulp or cotton fibres, or from a mixture of natural and manmade fibres. Further examples of topsheet materials are porous foams, apertured plastic films etc.

Preferably, coloured non-woven material is used for the topsheet 26, preferably having the same or substantially the same colour as at least the fabric layers of the article which define the colour of the outside of the article. The absorption zone, i.e. where the core is located, is preferably of a lighter colour as a result of the core, which may be entirely in relatively light colours, shining through the non-woven material of the topsheet 26.

Preferably, coloured material is also used for the backsheet 25, preferably the same colour as the topsheet 26.

The absorbent core 2 can be of any conventional kind. Examples of common absorbent materials used in absorbent cores are cellulosic fluff pulp, tissue layers, highly absorbent polymers (so called superabsorbents), absorbent foam materials, absorbent nonwoven materials or the like. It is common to combine cellulosic fluff pulp with superabsorbent polymers in an absorbent core.

It is conventional in absorbent articles to have an absorbent core 2 comprising layers of different properties with respect to liquid receiving capacity, liquid distribution capacity and storage capacity. The thin absorbent bodies, which are common in for example baby diapers and incontinence articles, often comprise a compressed mixed or layered structure of cellulosic fluff pulp and superabsorbent polymers. The size and absorbent capacity of the absorbent core may be varied to be suited for different uses such as for infants or for adult incontinent persons.

The absorbent core 2 may further include an acquisition distribution layer (not shown) placed on top of the primary absorbent body and which is adapted to quickly receive and temporarily store discharged liquid before it is absorbed by the primary absorbent core. Such acquisition distribution layers are well known in the art and may be composed of porous fibrous waddings or foam materials.

The layers or fabrics forming at least the top side of the core, e.g. the acquisition layer, are preferably of lighter colours, preferably at least lighter than the topsheet 26, for example L* values above 90, for example white or substantially white.

At least part of or the entire front and back portions 5 and 6 may be composed of an elastic laminate material 20. By the term "elastic" is meant that the material is capable of being extended under a force and then is capable of contracting back to or towards its initial length once the force is removed. The elastic laminate 20 used in the pant-type absorbent article may have an elasticity in the x-direction of the article of at least 30%, preferably at least 50%, more preferably at least 70%. The elastic laminate material 20 may also be elastic in the y-direction of the article. However the elasticity in the y-direction is preferably lower than in the x-direction.

Preferably the elastic laminate material 20 is also elastic in the y-direction of the article. However the elasticity in the y-direction is preferably lower than in the x-direction.

The term "non-elastic" refers to any material that does not fall within the definition of an "elastic" material given above.

The elastic laminate 20 may cover the entire article, including the core region 3 and the entire chassis region 4. However, in a preferred embodiment a part of the connecting portion 19 of the article is free from the elastic laminate material 20. The part of the connecting portion 19 that is free from the elastic laminate 20 includes the narrow part which is referred to as the crotch region 19a. The waist band 18 may or may not be free from the elastic laminate material 20. The waist band may comprise a nonwoven material that is elasticized by elongated elastic members 18a, such as elastic threads, contractably affixed between material layers, such as nonwoven materials. Ultrasonic welds, glue strings or the like, join the elastic laminate 20 to the elastic waist band 18.

The elastic laminate 20 preferably extends continuously laterally across the width of the front portion 5 between the first and second side edges 8 and 9 as well as continuously laterally across the width of the back portion 6 between the first and second side edges 11, 12. The elastic laminate 20 also extends continuously laterally across the width of the connecting portion 19 between the leg openings 16 and 17 in those parts of the connecting portion 19 where the elastic laminate 20 is present.

The elastic laminate 20 is composed of inner and outer layers of fibrous material 21 and 22, which form inner and outer fabrics of the article, and an elastic film 23 located between said fibrous layers. The elastic laminate 20 may also comprise one or more additional fibrous layers laminated to one or both of the first and second fibrous layers. Such additional fibrous layers may be present only in parts of the elastic laminate 20. Thus the elastic laminate 20 need not be identical all over its area, but may comprise different layers in different areas.

The inner and outer layers 21, 22 of the elastic laminate form portions of the inside and outside surface of the article. The material of these layers is preferably coloured, preferably has the same colour as the topsheet 26.

It is advantageous that the inner and outer fibrous layers are chosen so that they, in combination with the inner elastic film layer, give the material high resistance to puncture. They also provide a soft and cloth-like feel to the laminate. Examples of suitable materials are carded webs and spunbond materials. The basis weight of the fibrous material layers should be between 8 and 35 g/m2, preferably between 10 and 25 g/m2, more preferably between 12 and 25 g/m2. Examples of suitable polymers used in the fibrous materials are polyethylene, polyesters, polypropylene and other polyolefin homopolymers and copolymers. Natural fibres, for example cotton, may also be used as long as they provide the required properties. A mixture of polymers can contribute to a higher flexibility of the nonwoven layer, and through this, give the nonwoven material a higher elongation at maximum load. A mixture of polyethylene and polypropylene polymers has proved to provide good results in this respect. A mixture of fibers of different polymers is also possible.

At least one of the fibrous layers of the elastic laminate may be a creped nonwoven material. The creped nonwoven will increase the puncture resistance of the laminate puncture resistant and allow it to be subjected to the pulling and stretching forces that occur when putting on and taking off the pant article without breaking and tearing.

The middle layer is preferably an apertured elastic film 23 having a basis weight between 20 and 80 g/m2, preferably between 20 and 60 g/m2. The film may be of any suitable elastic polymer, natural or synthetic. Some examples of suitable materials for the elastic film are low crystallinity polyethylenes, metallocene-catalyzed low crystallinity polyethylene, ethylene vinyl acetate copolymers (EVA), polyurethane, polyisoprene, butadiene-styrene copolymers, styrene block copolymers, such as styrene/isoprene/styrene (SIS), styrene/butadiene/styrene (SBS), or styrene/ethylene-butadiene/styrene block copolymer. Blends of these polymers may also be used as well as other modifying elastomeric or non-elastomeric materials. One example of a suitable film is an apertured three-layer elastomeric film of PE-SEBS-PE.

It is further preferred that the elastic laminate 10 has a breathability (Water Vapour Transmission Rate) according to ASTM E96-00 Procedure D of at least 1500 g/m2 24h, preferably at least 3000 g/m2 24h.

The open area of the elastic film layer is preferably at least 5%, more preferably at least 8%. The open area is measured by image analysis methods and is defined as the sum of the hole area divided by the total area of the film sample.

In the following, methods for manufacturing elastic laminates 20 are described. In accordance with the present disclosure, coloured materials are used for the inner and/or outer fibrous layers of the laminate, in order to achieve a finished article with coloured inner and/or outer fabrics. Preferably, the same colour is used for both the inner and outer fibrous layers 21, 22.

One method for manufacturing an elastic laminate is described in WO 03/047488, wherein one spunbond layer is applied to the film, said film being in a tacky state and will thus bond to the spunbond layer, while the other spunbond layer is adhesively laminated to the film layer, using for example a pressure sensitive hot melt adhesive. Alternatively the laminate is manufactured according to a modified version of this known method, wherein the modification involves that the laminate is incrementally stretched (through intermeshing gears, IMG), to a point below the elongation at peak load of at least one of the non-elastic nonwoven layers to retain some strength for at least one of the nonwoven layers. The other layer may also be stretched to a point below its elongation at peak load, or to a point at which it will tear during stretching.

The method disclosed in WO 03/047488 involves stretching of the laminate above the point of failure of the fibrous material, so that the non-elastic layers break completely. Therefore, as described in WO 03/047488, the elongation of the laminate is not limited by the stretch modulus of the non-elastic material.

According to a modified method at least one, preferably both fibrous layers, which are bonded to the elastic film are not, in contrast to the method described in WO 03/047488, completely torn upon manufacture of a laminate. Selection of fibrous materials which have an elongation at maximum load greater than the elasticity of the elastic laminate allows the elastic film to stretch without being hindered by the fibrous layers. Such a selection also ensures that the fibrous layers contribute to the puncture resistance of the laminate, as they are not completely torn or broken during manufacture. Preferably the fibrous layers, or at least one of the fibrous layers has an elongation at maximum load that is at least 10% higher than the elasticity of the laminate. This is described in more detail in WO 2005/122985.

In an alternative embodiment the laminate 20 is manufactured by feeding a first fibrous layer in the form of a nonwoven web into a bonding nip and extruding a molten elastic film-forming polymer through a die into the nip. The first fibrous layer and the elastic film form a first laminate. In a second lamination step the film side of the first laminate is coated or sprayed with adhesive and is subsequently passed through a second bonding nip together with a second fibrous layer to form the laminate 20. The laminate is subsequently activated by subjecting it to incremental stretching by passing it through intermeshing gears, IMG.

In a further embodiment the inner layer 21 of fibrous material and the elastic film layer 23 form parts of a first elastic laminate that has been rendered elastic by incremental stretching and partial tearing of the inner layer of fibrous material and in which the first elastic laminate has been bonded to the outer layer 22 of fibrous material while in a stretched state. The resulting laminate will then be elastically stretchable.

In a still further embodiment the inner and outer layers 21, 22 of fibrous material have been bonded to the elastic film layer 23 while this is in a stretched state, so called stretch-bonding. The resulting laminate will be elastically stretchable.

The elastic laminate material 20 is preferably arranged as an outside coversheet material as well as inner coversheet material over at least part of the front portion 5, back portion 6 and connecting portion 19 of the chassis 4. The elastic laminate material may constitute the sole component of the chassis 4 in at least 20%, preferably at least 25%, more preferably at least 30% and most preferably at least 40% of the total surface area of the article, as seen in a flat state according to Figure 2 and 7.

No additional elasticized side panels joining the front and back portions 5 and 6 are needed when using the elastic laminate material 20.

The elastic laminate 20 and the backsheet 25 overlap in the outer parts of the core region 3, wherein the elastic laminate 20 is arranged on the garment facing side of the backsheet 25.

**The** absorbent assembly comprising the liquid impervious backsheet material 25, the liquid pervious topsheet material 26 and the absorbent core 2 enclosed therebetween, all of which components are described above, may be joined to the elastic laminate 20 of the front, back and/or connecting portions 5, 6 and 19 while this is held in a selectively stretched condition, so that gathers are present in the absorbent assembly at those points where it is joined to the front, back and/or connecting portions 5, 6 and 19.

As mentioned above the elastic laminate 20 may be absent in a substantial part of the connecting portion 19 of the article. A crotch panel material 24 may underlie at least part of the absorbent assembly on the garment-side thereof. The crotch panel material 24 may be of a non-elastic web material, although elastic materials may also be used. In case an elastic material is used as crotch panel material 24 it should be less elastic than the elastic laminate 20. Suitably, the crotch panel material is a nonwoven material. The crotch panel material 24 is joined to the elastic laminate 20 along seams 33 and 34.

Preferably the elastic laminate 20 is held in a stretched condition when joined to the non-elastic crotch panel material 24, wherein gathers are formed in the crotch panel material when the stretching force is released.

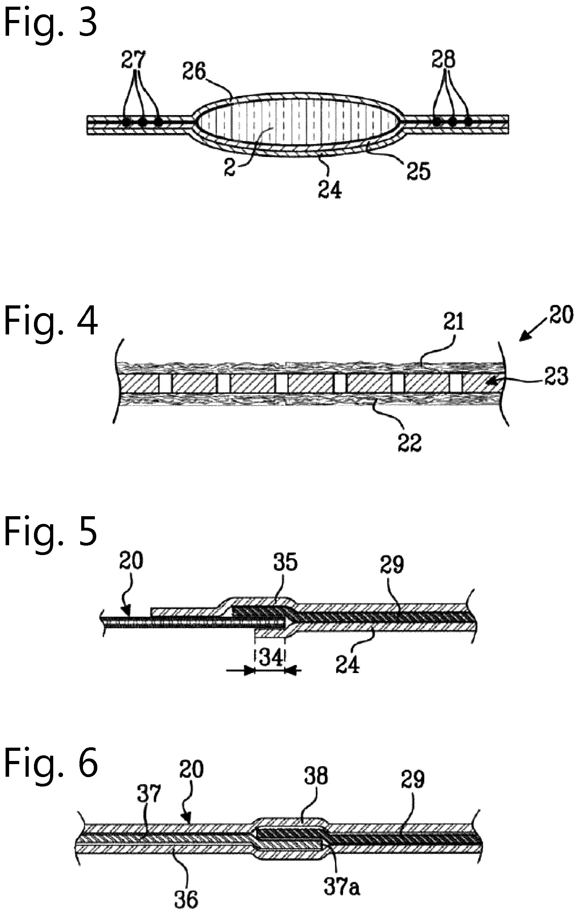

In an alternative embodiment illustrated in Fig. 6 there is no separate crotch panel material 24 joined to the elastic laminate 20 along seams, but the elastic laminate 20 is defined by an elastic film 37 laminated to only part of inner and outer nonwoven layers 36 and 38, wherein the transverse edge 37a of the elastic film 37 forms the boundary between the elastic laminate and the non-elastic crotch panel material. This will be described more in detail below.

Elongated leg elastic members such as elastic threads extend along part of the leg openings 16 and 17 in the connecting portion 19. In the embodiment disclosed in Fig. 2 the leg opening elastic members are divided in first elastic members 27 and 28 extending along the respective longitudinal edges of the crotch region 19a and second elastic members 29 and 30 extending along the edges of part of the leg openings 16 and 17 outside the area of the crotch region 19a. The area of the connecting portion 19 outside the crotch region 19a and that faces the front portion 5 is defined as the front part 19b of the connecting portion 19 and the area of the connecting portion 19 outside the crotch region 19a and that faces the back portion 6 is defined as the back part 19c of the connecting portion 19.

**The** first elastic members 27 and 28, which extend along the narrow part of the connecting portion 19 referred to as crotch region 19a provide for a sealing effect in the crotch part preventing leakage of body fluid.

In the embodiment disclosed in Fig. 7 leg elastic members 39, 40 extend continuously along part of the leg openings 16 including the crotch region 19a.

In the embodiments shown in Figs. 1-7 the elastic laminate material 20 covers more or less the entire front portion 5, the entire back portion 6 and part of the connecting portion 19, i.e. part of the leg opening area. In the embodiment disclosed in Fig. 2 the elastic laminate material 20 covers the entire front part 19b of the connecting portion 19 but only part of the back part 19c of the connecting portion 19. The part of the leg opening area in the connecting portion 19 covered by the elastic laminate 20 is elastic also in the absence of additional leg elastics.

In the embodiment shown in Fig. 2 the second elastic members 29 and 30 extend from an area adjacent the point 16a, 17a in the connecting portion 19 where the first elastic members 27 and 28 terminate and where the width of the article increases abruptly. The second elastic members 29 and 30 are only arranged in the back part 19c of the connecting portion 19 that is facing the back portion 6, while no second elastic members 29 and 30 are arranged in the front part 19b of the connecting portion 19.

The second elastic members 29 and 30 do not extend all the way to the side seams 13 and 14 but terminate in the leg openings 16 and 17 at a distance from the respective side seam 13 and 14 thus leaving an area 31 and 32 corresponding to a peripheral length p of at least 50 mm, preferably at least 75 mm and more preferably at least 100 mm along the respective leg opening 16 and 17 where the leg elastic members 29 and 30 are absent. The peripheral length p is measured along the periphery of the respective leg opening 16 and 17 and is measured to the inner edge of the respective side seam 13 and 14. When more than one elongated elastic member is present in the leg elastics (which normally is the case) the peripheral length (p) is measured from the elastic member where said length is shortest. The length p is measured in a flat, non-contracted state of the article as illustrated in Fig. 2. Said flat, non-contracted state of the article corresponds to the degree of stretching the elastic laminate has during the production process, when attaching non-elastic material components thereto.

The connecting portion 19 in the area adjacent the leg openings 16 and 17 where the leg elastic members 29 and 30 are absent is composed of the elastic laminate material 20 along at least 80%, preferably at least 85% and more preferably at least 90% of the peripheral length p.

As described above a crotch panel material 24 is arranged in the crotch region 19a of the connecting portion 19 and is joined to the elastic laminate 20 of the connecting portion 19. The crotch panel material 24 is preferably non-elastic. The leg elastic members 27-30 extend along at least part of the leg openings 16 and 17 in the connecting portion 19 defined by the crotch panel material 24. The leg elastic members 27-30 preferably extends along at least 90% of the part of the leg openings 16 and 17 which are located in said crotch panel material 24, wherein said length is measured along the periphery of the respective leg opening.

The leg elastic members 27-30 may extend a certain distance into the part of the leg openings 17 and 18 defined by the elastic laminate 20, or they may end in the boundary between the elastic laminate 20 and non-elastic crotch panel material 24, such as in a seam 34 joining the elastic laminate 20 to the crotch panel material 24. Preferably the leg elastic members 27-30 overlaps the elastic laminate 20 not more than 20 mm and more preferably not more than 15 mm.

A cover strip 35, preferably a nonwoven material, may be laminated on the wearer-facing side of the article covering the leg elastic members 29 and 30. The cover strip 35 preferably extends the entire width of the article. The cover strip 35 forms part of the elastic laminate 20 so that the part of the elastic laminate 20 to which the cover strip 35 is laminated maintains at least a substantial part of its elastic properties.

The leg elastics 29 and 30 retract the web material to which they are attached causing wrinkles, which can be seen through the clothing. The arrangement of having leg elastics 29 and 30 that do not extend all the way to the side seams 13 and 14 results in smoother leg openings with less wrinkles and a more discrete article. Since at least a major part of the leg openings where the leg elastics are absent is composed of the elastic web material 20 there will be a certain sealing effect also in the area where the leg elastics are absent.

In an alternative embodiment illustrated in Fig. 6 there is no separate crotch panel material 24. Instead a base layer nonwoven 36 extends over the front, back and crotch portions 5, 6 and 19 and forms an outer coversheet material thereof. An elastic film 37 is laminated to the base layer nonwoven 36 in the front and back portions 5 and 6. A cover layer nonwoven 38 is laminated to the opposite side of the elastic film 37, and forms an inner coversheet of the article. The base layer nonwoven 36, the elastic film 37 and the cover layer nonwoven 38 forms an elastic laminate 20 as described above. The base layer nonwoven 36 and the cover layer nonwoven 38 are preferably per se non-elastic, wherein the article is elastic only in those parts where the elastic film 37 is present. The base layer nonwoven 36 and the cover layer nonwoven 38 form the crotch panel material in this embodiment.

The elastic film 37 extends preferably transversely across the width of the front portion 5 between the first and second side edges 8, 9, transversely across the width of the back portion 6 between the first and second side edges 11, 12 and transversely across part of the connecting portion 19 between the leg openings 16 and 17. The elastic film 37 has a transverse edge 37a at its end facing the crotch region 19a of the connecting portion 19.

Elongated leg elastic members 29 and 30 are attached between the base layer nonwoven 36 and the cover layer nonwoven 38 along part of the respective leg opening 16 and 17 at least in areas where the elastic film 37 is absent, in a corresponding manner as disclosed with respect to the embodiment described above. Preferably the leg elastic members 29 and 30 extend at least along 80%, preferably at least along 85% and more preferably along at least 90% of the part of leg openings 16 and 17 located in said area where the elastic film 37 is absent, wherein said length is measured as the peripheral length p along the respective leg opening. Preferably the leg elastic members 29 and 30 overlap the elastic film 37 not more than 20 mm and more preferably not more than 15 mm.

In the embodiment disclosed in Fig. 7 the leg openings 16 and 17 have a different curvature than in the embodiment disclosed in Fig. 2 and leg elastic members 39 and 40 extend continuously along part of the leg openings 16 and 17 including the crotch part 19b of the connecting portion 19. In other words this embodiment is similar to the ones described above.

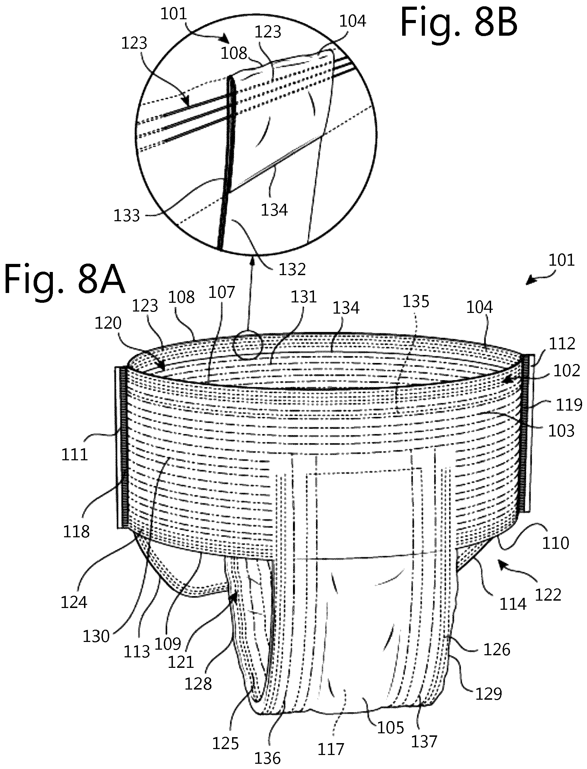

A second set of embodiments of absorbent articles according to the present disclosure will be described with reference to Figs. 8-13, in particular threaded type pant articles. Fig. 14a and 14b schematically show the inside and outside of such articles.

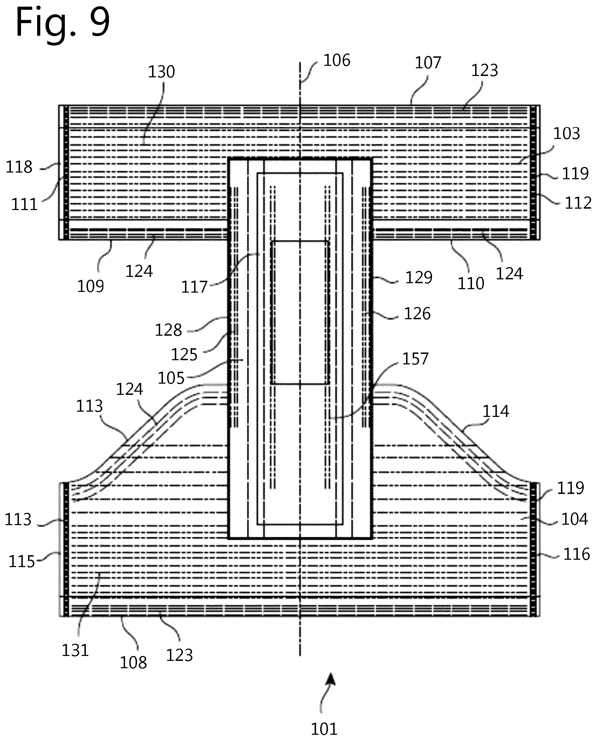

With reference to Figure 8a of the drawings, there is shown an embodiment of a disposable pant-type absorbent article 101 illustrated in an assembled and ready-to-use state. The same absorbent article 101 is also shown in Figure 9, but in a condition in which it is laid out flat and as viewed from above in order to show its main components. Furthermore, Figure 8b is a perspective view of an enlarged section of a part of the absorbent article 101, as will be described in greater detail below.

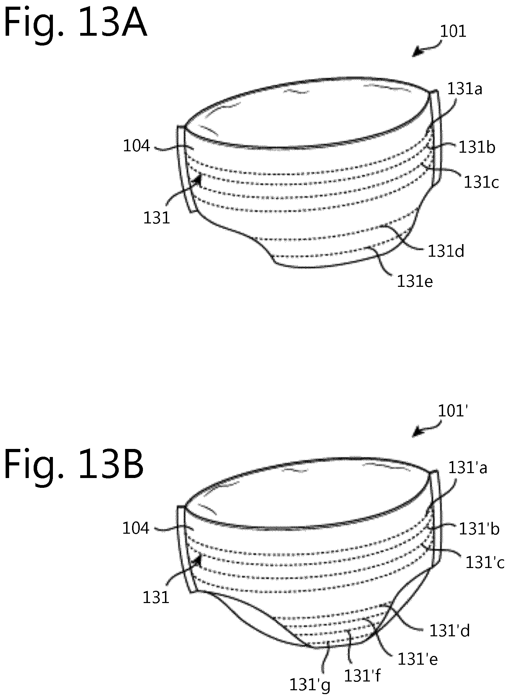

The article 101 shown in Figures 8-9 is not intended to describe a specific male or female model, but is used to describe the general principles of an article which can be either suited for male or female users. However, Figures 13a and 13b, which will be described in detail below, and indicate the difference between male and female articles.

With reference to Figure 8a and Figure 9, the pant-type absorbent article 101 is for example in the form of a pant diaper, a sanitary pant or an incontinence pant adapted for use by a baby, child or adult, male or female user. The pant-type absorbent article 101 according to Figure 8 comprises a single-piece chassis 102 having a front portion 103, a back portion 104, a crotch portion 105 connecting the front and back portions 103, 104, and a centre line 106 (see Figure 9) in the longitudinal direction of the article. The absorbent article 101 has a longitudinal direction, a transverse direction and a thickness direction.

The front portion 103 has a waist edge 107, a pair of leg edges 109, 110 and a pair of side edges 111, 112. Furthermore, the back portion 104 has a waist edge 108, a pair of leg edges 113, 114 and a pair of side edges 115, 116.

As mentioned above, the absorbent article 101 comprises a crotch portion 105, which comprises an absorbent body 117 located mainly in said crotch portion 115. The absorbent body 117 may be manufactured separately from the chassis 102 and inserted and fastened to the chassis 102 at a suitable manufacturing step. This process will be described in greater detail below.

The side edges 111, 112 of the front portion 103 are attached to the opposite side edges 115, 116 of the back portion 104 by means of permanent or re-closable side connections 118, 119 such as side seams, hook and loop fasteners, adhesive fasteners, or the like, in order to at least partly define a waist opening 120 and a pair of leg openings 121, 122.

A first elastic element in the form of an elastic waist component 123 is fastened to the chassis 102 at least partly along the waist edges 107, 108 forming part of the front portion 103 and the back portion 104. The purpose of the elastic waist component 123 is to provide the absorbent article 101 with a good fit around the waist of the user wearing the article. The elastic waist component 123 is fastened relatively close to the waist edges 107, 108, around the waist opening 120.

Furthermore, a second elastic element in the form of an elastic leg component 124 is fastened to the chassis 102 at least partly along the leg edges 109, 110 of the front portion 102 for the purpose of providing the absorbent article 101 with a good fitting around the legs of the user wearing the article. The elastic leg component 124 is fastened relatively close to the leg edges 109, 110.

As shown in particular in Figure 9, the elastic leg component 124 forms a straight line in the front portion 103 and has a curved configuration in the back portion 104.

Furthermore, a first absorbent body elastic 125 and a second absorbent body elastic 126 are arranged along the crotch portion 105. The first absorbent body elastic 125 is arranged along a first crotch edge 128 whereas the second absorbent body elastic 126 is arranged along a second crotch edge 129. In particular, the first absorbent body elastic 125 and the second absorbent body elastic 126 are arranged relatively close to the longitudinal crotch edges 128, 129. In a similar manner, the elastic waist component 123 and the elastic leg component 124 are also arranged relatively close to the waist edges 107, 108 and the leg edges 109, 110, respectively, as shown in Figure 8 and Figure 9.

If the elastic leg component 124 and the elastic waist component 123 are fastened at a location close to the leg and waist edges 107, 108, 109, 110, respectively, less non-elasticised web material is available at the leg and waist edges such that less frills is created along said edges. This is an advantage, since a large amount of material at the leg edges may be perceived as uncomfortable by a user and may give the user an impression that the article is not similar to conventional underwear.

Having the elastic leg feature 124 positioned closer to the leg edge 109, 110 may also result in an absorbent article 101 having an improved fit which corresponds to the shape of the legs of the user. It is thus desirable to provide an elasticised leg edge 109, 110 of the front and back portion 103, 104 that has a more cuff like appearance with less frills, thereby providing the absorbent article 101 with an appearance more similar to cloth underwear.

Furthermore, as shown in Figure 8a and Figure 9, the absorbent article 101 comprises a front elastic component 130 and a back elastic component 131 which are both based on a number of elastic threads mounted at a certain distance from each other in a generally parallel manner around the article 101, i.e. the region of the belly and the backside of the user. The purpose of these elastic components 130, 131 is to contribute to a good fit and comfort for the wearer of the article 101. In particular, the configuration of the elastic threads can be adapted to the male and female anatomy and the need for a suitable fit and comfort for male and female users of the article 101.

The positioning of the elastic threads and also the elastic properties of the elastic threads can be individually adapted so as to provide a configuration of the back elastic component 131 and front elastic component 130 which is arranged so as to individually fit the male and female anatomy, respectively. This will be described in greater detail below with reference to Figure 13a and Figure 13b. More precisely, the positioning of the elastic threads refers to the manner in which the threads are laid out, in a geometric sense, along the absorbent articles in the array and also the distance between any two adjacent elastic threads. According to a further embodiment, the number of elastic threads used can also be chosen so as to provide a configuration of the back elastic component 131 and front elastic component 130 which is arranged so as to individually fit the male and female anatomy, respectively.

Furthermore, and as mentioned above, it can be noted that a process of fastening the elastic waist component 123 and the elastic leg component 124 close to an edge of a web material, i.e. in this case close to the waist edges 107, 108 and the leg edges 109, 110, respectively, is difficult due to the manufacturing tolerances of the production line. The provision of elastic elements along the edges of the article, i.e. along the waist, legs and crotch edges, contributes to a modern and well-fitting absorbent article such as an incontinence article. Such elastic elements are normally provided with a number of elastic threads which are arranged along a waist edge, a leg edge and two crotch edges.

A production process for a pant-type absorbent article operates at a high rate and such a fully automatized manufacturing line needs to have a certain tolerances. If the elastic threads of the elastic components are positioned too close to the corresponding edges, there is a risk that the threads may actually be laid and positioned outside the edges. Since glue is normally applied to the threads, there is a risk for production interruption if the elastic threads are erroneously positioned outside the actual edges of the article.

With further reference to Figure 9 and also Figure 8b, there is provided an embodiment in which the elastic waist component 123, the elastic leg component 124 and the elastic absorbent body components 125, 126 are fastened by means of a folding arrangement of the article 101 in question. The principles for this folding arrangement are shown in Figure 8b, which shows an enlargement of a small section of the upper part of the back portion 104, more precisely a section of the absorbent article 101 close to the waist edge 108 of the back portion 104.

In a manner which is conventional as such, the absorbent article 101 comprises a liquid permeable topsheet 132, i.e. a sheet which is intended to face the user of the article 101, and a liquid impermeable backsheet 133, i.e. a sheet which is placed so as to face the garment worn by the user. Generally, the liquid permeable topsheet 132 comprises or consist of a nonwoven material. The topsheet material may further be composed of tow fibres, porous foams, apertured plastic films and similar materials. The materials suited as topsheet materials should be soft and non-irritating to the skin and should be readily penetrated by body fluid, e.g. urine or menstrual fluid, and display low rewetting properties.

Preferably, coloured non-woven material is used for the topsheet 132, preferably having the same or substantially the same colour as at least the fabric layers of the article which define the colour of the outside of the article. The absorption zone, i.e. where the core is located, is preferably of a lighter colour as a result of the core, which may be entirely in relatively light colours, shining through the non-woven material of the topsheet 132.

Preferably, coloured material is also used for the backsheet 133, preferably the same colour as the topsheet 133.

Furthermore, the liquid impermeable backsheet 133 may consist of a thin plastic film, e. g. a polyethylene or polypropylene film, a nonwoven material coated with a liquid impervious material, a hydrophobic nonwoven material which resists liquid penetration or laminates of plastic films and nonwoven materials. The backsheet material may be breathable so as to allow vapour to escape from the absorbent article, while still preventing liquids from passing through the backsheet 133 material.

According to various embodiments, the materials which can be used for manufacturing the backsheet 133 include thin and flexible fluid impermeable plastic films, or fluid impermeable nonwoven materials, fluid impermeable foams and fluid impermeable laminates.

Furthermore, the backsheet 133 is formed by a single layer, and can alternatively be formed by a multi-layered structure, i.e. a laminate, wherein at least one layer is fluid impermeable. Furthermore, the backsheet 133 can optionally be elastic in either direction. According to further embodiments, the backsheet 133 may be breathable, implying that air and vapor may pass through the backsheet. Furthermore, the backsheet 133 may optionally have an outer, garment-facing surface of a textile material such as nonwoven.

The absorbent core 117 can be formed by a single layer consisting of fibres of cellulosic fluff pulp. According to alternative embodiments, the absorbent core 117 can be made up of any suitable absorbent or fluid-absorbing material as known in the art, for example foam, fiber waddings and similar materials.

Furthermore, the absorbent core 117 may consist of a mixture of cellulosic fluff pulp and a suitable amount of superabsorbent particles. Such superabsorbent material is well known in the field of absorbent articles, and is constituted by a water-swellable and water-insoluble material which is capable of absorbing large quantities of fluid upon formation of a hydrogel. Normal superabsorbent materials are capable of absorbing fluids of at least 10 times its own weight.

According to further embodiments, the absorbent core 117 may further incorporate components for improving the properties of the absorbent core. Some examples of such components are binder fibers, fluid-dispersing materials, fluid acquisition materials, etc. as known in the art. The absorbent core 117 may also be a homogeneous structure or may be a layered structure with laminates of the same or different materials. The absorbent layers may have uniform thickness or may vary in thickness in different parts of the layers.

The layers or fabrics forming at least the top side of the core are preferably of lighter colours, preferably at least lighter than the topsheet 132, for example L* values above 90, for example white or substantially white.

The topsheet 132 and backsheet 133 may be connected to each other for example by adhesive bonding, gluing or welding by heat or ultrasonic. The topsheet 132 and/or the backsheet 133 may further be attached to the absorbent body by any method known in the art, such as adhesive, heat-bonding etc.

According to an embodiment, the topsheet and backsheet, herein also called inner and outer fabrics, in the portions forming the front portion 103 and the back portion 104 may be of different type than the topsheet and backsheet in the crotch portion 105. In the latter case, it is suitable with a fluid-permeable topsheet and a fluid-impermeable backsheet (as described above) since the absorbent article 101 must have absorbent properties in the crotch portion 105. However, in the parts forming the front portion 103 and the back portion 104, both the topsheet and the backsheet may be for example liquid-impermeable, since these parts of the absorbent article 101 generally do not need to have absorbent properties. The fabrics or materials used for this topsheet and backsheet in the front and back portions 103, 104 are preferably coloured, preferably in the same colour as the topsheet 132 in the crotch portion.

According to the embodiment shown in Figure 8b, the backsheet 133 is configured so that it can be folded along the waist edge 108 of the back portion 104 and over the topsheet 132. In this manner, the elastic waist component 123 is at least partly enclosed between the topsheet 132 and the backsheet 133. More precisely, the elastic waist component 123 is positioned between the backsheet 133 and the topsheet 132, and the backsheet 133 is then folded over the topsheet 132. In this manner, an edge 134 of the backsheet 133 is defined along the inside of the absorbent article 101, i.e. facing the user of the article.

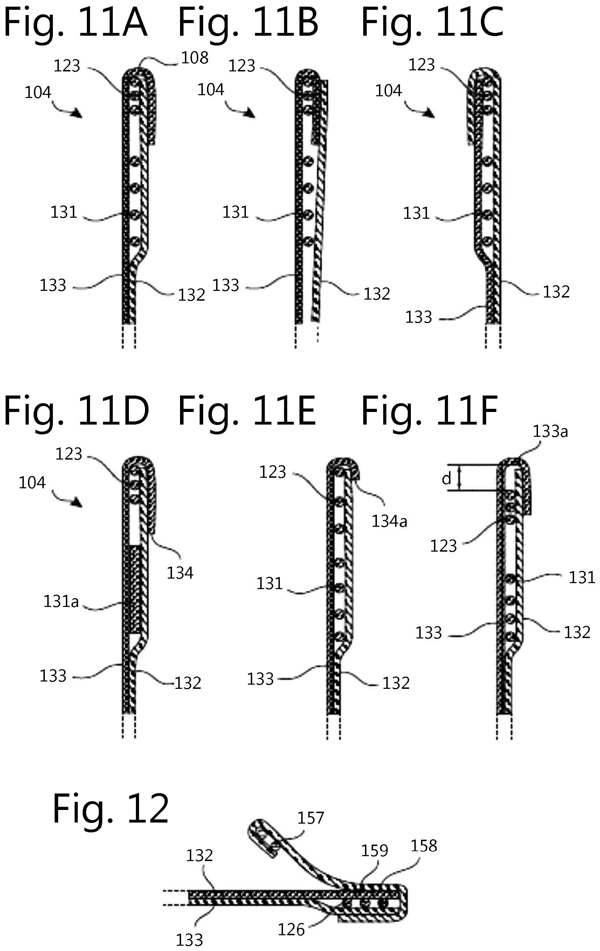

The embodiment shown in Figure 8b is configured so that the elastic waist component 123 is fully enclosed by means of the topsheet 132 and the folded backsheet 133. However, according to other embodiments (as will be described below with reference to Figure 11e), the backsheet 133 and the elastic waist component 123 may be dimensioned and configured so that the backsheet 133 is folded in a manner so as to enclose only a part of the elastic waist component 123.

A similar folding process is carried out also as regards the front portion 103, so that the backsheet 133 forms a fold defining an edge 135 (see Figure 8a) along the inside of the absorbent article 101. Furthermore, a similar folding process is carried out also as regards the crotch portion 105, so that a fold is formed with a first edge 136 and a second edge 137 (see Figure 8a) along the absorbent body 117 in the crotch portion 105. Also, a similar folding process is carried out also so as to enclose the elastic leg component 124 along the leg edges 109, 110.

The purpose of the folding procedure as described above is to allow the elastic elements, i.e. the elastic waist component 123, the elastic leg component 124 and the elastic absorbent body components 125, 126, to be positioned very close to the corresponding edge of the absorbent article 101. This means that the absorbent article 101 can be manufactured in a manner with so that it resembles an ordinary undergarment which has an optimized waist elastic function and which is convenient to wear. By positioning the elastic elements very close to each edge of the article, the amount of unelasticized web material which otherwise may occur along the edges can be avoided. In summary, the absorbent article 101 will be more similar in look and feel to regular underwear, while still offering sufficient protection against urine leakage. Furthermore, in an array of gender-specific absorbent articles, an elastic waist component, an elastic leg component and an elastic absorbent body component can be adapted in a suitable manner in article intended to be worn by a male and female user, respectively.

Consequently, the backsheet 133 or the topsheet 134 is folded along the waist edges 107, 108, leg edges 109, 110 and crotch edges 128, 129 so as to enclose each corresponding elastic element 123, 124, 125, 126. Certain alternative embodiments will be further described below.

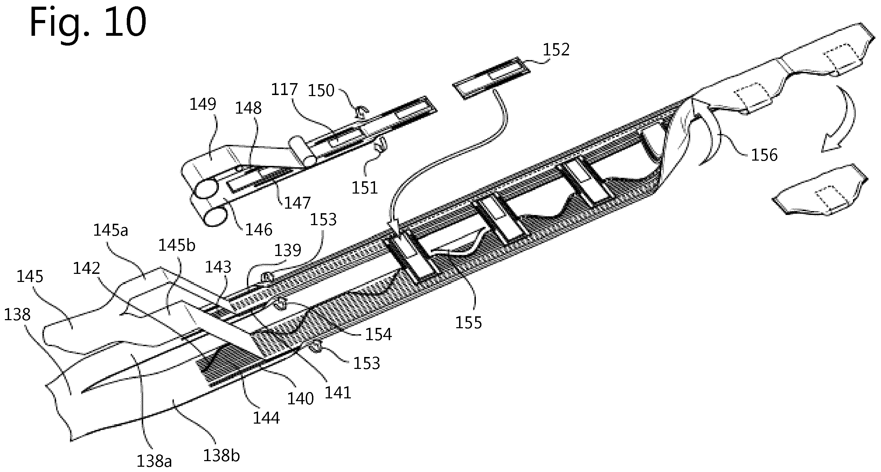

**The** positions and the elastic properties of at least the elastic threads forming the front elastic component 130 and the back elastic component 131 can be varied in order to provide articles which are adapted to the male and female anatomy and to other requirements regarding male and female absorbent products. An example embodiment of a manufacturing line for a pant-type absorbent article 101 is schematically illustrated in Figure 10.

**A** first continuous sheet 138 of web material is supplied and is also divided, in a lengthwise manner, so as to form a first web section 138a and a second web section 138b. The first web section 138a forms the basis of a backsheet for the front portion 103 (see Figures 8 and 9) of the absorbent article 101, whereas the second web section 138b forms the basis of a backsheet for the back portion 104.

A plurality of strips of elastic material are attached to the first and second web sections 138a, 138b in a tensioned state. More precisely, a first strip 139 and a second strip 140 of elastic material form the basis of the elastic waist component 123, and a third strip 141 and a fourth strip 142 of elastic material form the basis of the elastic leg component 124. Also, a fifth strip 143 and a sixth strip 144 of elastic material form the basis of the front elastic component 130 and the back elastic component 131, respectively.

The strips 139, 140, 141, 142, 143, 144 of elastic material may be glued or otherwise fastened to the continuous sheets 138a, 138b of web material, and said strips are intended to form an elastic web feature of the absorbent article 101 as described above with reference to Figures 8-9.

Next, a further continuous sheet 145 of web material is provided and is split longitudinally in order to form a third web section 145a and a fourth web section 145b. The third web sections 145a forms the basis of a topsheet for the front portion 103 whereas the fourth web section 145b forms the basis of a topsheet for the back section 104.

The third web section 145a and the fourth web section 145b are joined to the first web section 138a and the second web section 138b, respectively, in order to form a laminated product having the strips 139, 140, 141, 142, 143, 144 of elastic material sandwiched between the first web section 138a and the third web section 145a, and also between the second web section 138b and the fourth web section 145b. The second sheets may be attached to each other by ultrasonic bonding, welding, adhesive, embossing, mechanical fastening, or the like. The attachment of the above-mentioned web sections and elastic strips is here described as being performed in consecutive steps but these steps are typically performed in a single step.

In order to form the crotch portion 105 (see Figure 8a) with its absorbent core 117, a third continuous sheet 146 of web material is provided and forms the basis of a backsheet for the crotch portion 105. The absorbent core 117 is then laid out on the third continuous sheet 146. Also, a seventh strip 147 and an eighth strip 148 of elastic material are also laid out on the third sheet 146 of web material. The seventh strip 147 of elastic material forms the basis of the first absorbent body elastic 125 (see Figures 8a and 9), whereas the eighth strip 148 of elastic material forms the basis of the second absorbent body elastic 126.

Next, a fourth continuous sheet 149 of web material is provided and is joined to the third continuous sheet 146, suitably in a manner which is similar to that described above with reference to the first web section 138a, the second web section 138b, the third web section 415a and the fourth web section 145b. During this process, the seventh strip 147 and eighth strip 148 of elastic material, as well as the absorbent core 117, are sandwiched between said third continuous sheet 146 of web material and said fourth continuous sheet 149 of web material.

In order to adapt the manufacturing process to a male-type article and a female-type article, respectively, the process can be modified by choosing suitable elastic material (i.e. for the strips 139, 140, 141, 142, 143, 144 of elastic material) to be included in the articles. Such selections of elastic material according to dimensions, brand, elastic properties and other parameters must be implemented in the manufacturing process in an efficient manner generally without any interruptions or time-consuming modifications.

A folding procedure is next carried out so as to fold the edges of the crotch portion 105 and form the edges 136, 137 on the crotch portion 105. This folding operation is indicated in a simplified manner with the arrows 150 and 151 in Figure 10. Similarly, folding of the front portion 103 and the back portion 104 is indicated in Figure 10 with arrows 153, 154 in a simplified manner. This folding operation corresponds to that which is shown in Figure 8b.

In a further manufacturing step, the web formed by means of the third sheet 146 of web material, the fourth sheet 149 of web material 149 and the absorbent core 117, is cut into individual pieces 152, each of which forms the above-mentioned crotch portion 105 which is subsequently attached to the web formed by the first and second web sections 138a, 138b and the third and fourth web sections 145a, 145b. In this regard, the crotch portions 105 are laid out at a predetermined distance so as to bridge the front portion 103 and the back portion 104 and to form the basis of the finished absorbent article. As shown in Figure 10, a piece 152 which forms a crotch portion 105 is laid out in a transversal direction in relation to the webs forming the front portion and the back portion.

The crotch portion 105 may be attached to the chassis using any known fastening technology, such as ultrasonic bonding, welding, adhesive, embossing, mechanical fastening, or the like. In this manner, a complete chassis is formed for the article 101 in question.

In a subsequent manufacturing step, leg openings 155 are cut out of the laminated web material forming the chassis of finished absorbent articles. The cutting may be performed by any type of suitable cutting equipment (not shown in Figure 10), such as rolling cutting using two opposite rollers.

Next, the first and fourth web sections 138b, 145b are folded to form the final product, such that the first web section 138b becomes a backsheet of the chassis and the fourth web section 145b becomes the topsheet of the chassis. This folding is shown with an arrow 156 in Figure 10. After for example welding of side seams, the continuous assembly of products is cut into individual absorbent articles by means of cutting equipment (not shown in Figure 10).

Figures 11a-f show cross-sectional views of alternative embodiments of the structure forming the back portion 104. Figure 11a shows an embodiment in which the elastic element 123 is sandwiched between an inner side of the backsheet 133 and an inner side of the topsheet 132. The above-mentioned back elastic component 131 is also shown in Figure 11a. Also, the backsheet 133 is folded over an outer side of said topsheet 132 so as to enclose the elastic element 123 and so as to define the edge 134. This procedure corresponds to the embodiment shown in Figure 8b and Figure 3.

Furthermore, Figure 11b shows an alternative embodiment in which the elastic element 123 is enclosed and covered within a fold which is defined by the backsheet 133. Subsequently, the topsheet 132 is attached to said backsheet 133, suitably by gluing.

Figure 11c shows a further alternative embodiment in which the elastic element 123 is sandwiched between an inner side of the backsheet 133 and an inner side of the topsheet 132, and wherein the topsheet 132 is then folded over an outer side of said backsheet 133 so as to enclose the elastic element 123.

Figure 11d shows a further alternative embodiment which generally corresponds to the embodiment shown in Figure 11a, but having a back elastic component 131a which is in the form of a relatively thin strip manufactured from an elastically stretchable film. As an example, a suitable thermoplastic elastomer can be used for such a stretchable film.

Furthermore, Figure 11e shows a further alternative embodiment which generally corresponds to the embodiment shown in Figure 11a but which shows a configuration in which the backsheet 133 is folded in a manner so that it partly encloses the elastic element 123. Consequently, this embodiment is arranged with a backsheet 133 and an elastic element 123 having other dimensions and configurations than the embodiment shown in Figure 11a, so that the edge 134 is closer to the waist edge 108 (see also Figure 8b) as compared with the embodiment in Figure 11a. Even though the elastic element 123 is only enclosed partly by the folded portion of the backsheet 133, the article can be designed in a manner which is similar to regular underwear while still providing relevant protection against incontinence and also sufficient comfort and fit.

Variations of the embodiments shown in Figure 11d and 11e but where the topsheet and backsheet are folded as in Figure 11b and Figure 11c, respectively, are also possible.

Figure 11f shows an embodiment in which the elastic element 123 is positioned at a certain distance d from the inside of the fold 133a which is defined by the backsheet 133. According to embodiments, the distance d from the inside of the fold 133a is less than 10 mm, preferably less than 5 mm, and most preferably less than 3 mm, in order to provide an absorbent article 101 which is similar to regular underwear while still offering sufficient protection against urine leakage.

Furthermore, Figure 12 shows a cross-sectional view of an embodiment involving the crotch section 105 and in particular showing the second absorbent body elastic 126 (see also Figure 8a and Figure 9). According to this embodiment, the crotch portion comprising a further web material 158 which is folded over the laminate which is defined by the topsheet 132 and the backsheet 133. In this manner, the second absorbent body elastic 126 is enclosed. According to a further embodiment, the crotch portion 105 can be equipped with so-called standing gathers comprising elastic elements 157 which are enclosed by a section of the further web material 158 which is attached to the topsheet 132 by means of adhesive 159 or another suitable fastening means. A similar arrangement can be made as regards the first absorbent body elastic 125 (see Figure 8a and Figure 9).

Figure 13a and Figure 13b are perspective views, as regarded from the rear of an absorbent article 101, 101', wherein Figure 13a shows a first configuration of an embodiment of the absorbent article 101 and Figure 13b shows a second configuration of an embodiment of the absorbent article 101'. The first configuration describes a male type of absorbent article 101, whereas the second configuration describes a female type of absorbent article 101'.

As indicated in Figure 13a, the article 101 is provided with a back elastic component 131 which according to an embodiment comprises an upper section with a plurality of elastic threads 131a, 131b, 131c etc., which are mounted along the article 101 at a predetermined distance from each other, and also a lower section with a further plurality of elastic threads 131d, 131e, which are also mounted at a predetermined distance from each other. The elastic threads are mounted during the manufacturing process, as described above with reference to Figure 11, in a generally parallel manner in the back portion 104, i.e. around the region of the article 101 which corresponds at least to the backside of the user. The distance between the elastic threads 131a-e and the extension of said threads 131a-e are chosen so as to suit a male user, i.e. adapted for the male anatomy and requirements regarding style, cut, comfort and fit for a male user. Also, the elastic properties of the elastic threads 131a-e, in particular as regards the dimensions, elastic force and choice of material, are selected in a corresponding manner to suit a male user of the article 101.

Referring now to Figure 13b, which is an article 101' suitable for a female user, it can be seen that this article 101' has a design which is similar to the article 101 shown in Figure 13a, i.e. including a back elastic component 131' comprising an upper section with a plurality of elastic threads 131'a, 131'b, 131'c etc. which are positioned in the same manner as the upper section shown in Figure 13a. However, the back elastic component 131' comprises a lower section which is different from the lower section shown in Figure 13a. More precisely, the lower section of the back elastic component 131' in Figure 13b has a higher number of elastic threads 131'd, 131'e, 131'f, 131'g than the lower section shown in Figure 13a. Also, the threads 131'd, 131'e, 131'f, 131'g in Figure 13b may be of different type than the threads shown in Figure 6a in order to adapt the article 101' according to Figure 13b for the female anatomy and also requirements regarding style, cut, comfort and fit suitable for a female user.

Also, although not visible in Fig. 13b, the elastic threads 131'd, 131'e, 131'f, 131'g of the lower section in Figure 13b may also have elastic properties - i.e. as regards the dimensions, elastic force and choice of material - which are suitable for a female user and which properties consequently are different than those of the corresponding elastic threads 131d, 131e shown in Figure 13a.

**The** male and female absorbent articles 101, 101' may differ as regards various features, for example:

- the number of elastic threads used in the back elastic components 131, 131';
- the elastic properties of the elastic threads 131, 131';
- the positioning of the threads and the distance between adjacent threads.

For example, the absorbent article 101 in Figure 13a may have a lower section with two elastic threads, whereas the corresponding lower section in the absorbent article 101' shown in Figure 13b may have four elastic threads.

Combinations of different features can be made in order to meet the requirements for male and female absorbent products.

Furthermore, according to an embodiment, a male article 101 (see Figure 6a) may comprise a number of elastic threads of approximately 540 dtex, whereas a female article 1' (see Figure 13b) may comprises a number of elastic threads of approximately 800 dtex, where dtex represents a unit of the linear density of a continuous filament or yarn of the corresponding elastic thread. This means that the lower section of the female article 101' is configured so as to provide a higher elastic force - as indicated by means of arrows in Figure 13b - to pull the lower section of the article 101' together to fit a female wearer.

Furthermore, although not shown in Figures 13a and 13b, it should be noted that the upper sections of the male and female articles 101, 101' also may have different elastic properties, i.e. in a similar manner as the lower sections.

Figure 13a and Figure 13b indicate that the articles 101, 101' for male and female users, respectively, are slightly different in cut and design. It should be noted that the above-mentioned adaptations for male and female users, respectively, are made without departing from the goal of an efficient manufacturing process as discussed above.

According to an embodiment, a manufacturing process in which a change between production of a male and a female product must be carried out can be made by simply switching the elastic threads adapted for a male article for other elastic threads (having other dimensions or elastic properties) adapted for a female article. Alternatively, the distance between the threads can be changed during such an operation. This also means that the leg opening of the articles can be cut in a similar manner, and with the same equipment, for both male and female articles.

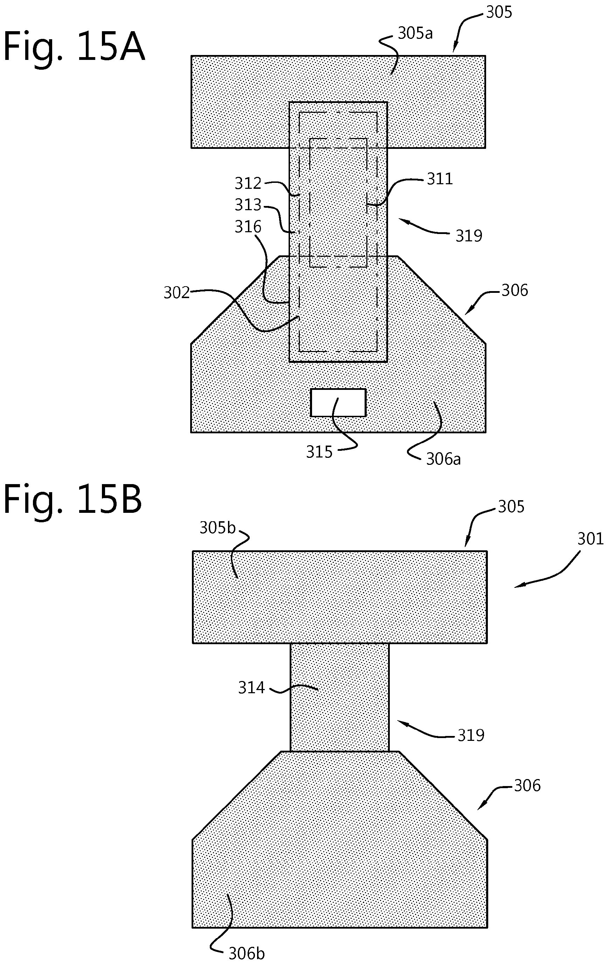

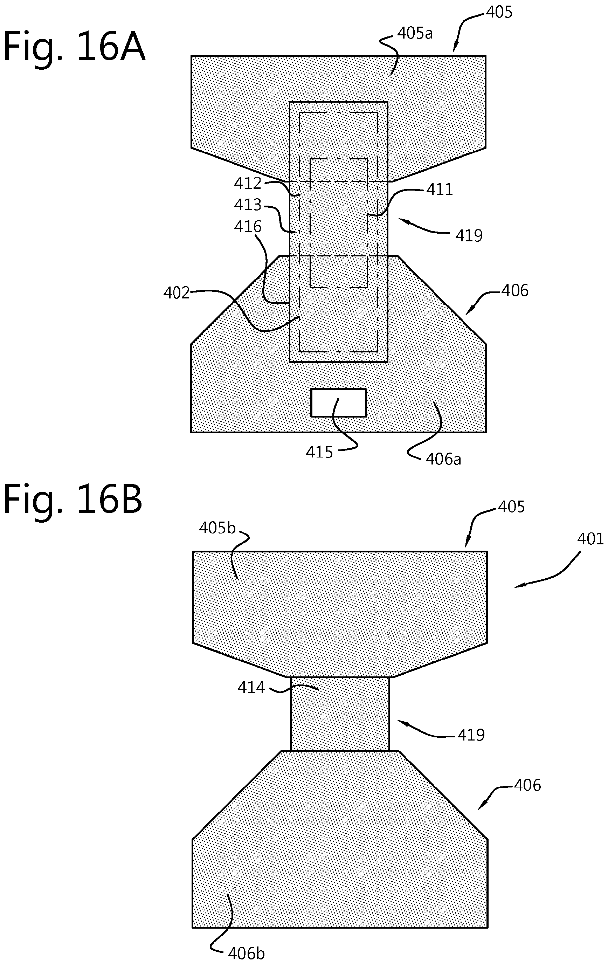

Figs. 14-16 schematically show an embodiment of an array according to the present disclosure, comprising a first absorbent pant article 201 (fig. 14), a second absorbent pant article 301 (fig. 15) which is structurally the same as the first article but has a different colour, and a third absorbent pant article 301 (fig. 16) which is structurally different from the first article and has the substantially the same colour as the second article (i.e. the fabrics have mutual colour differences ∆E*ab less than 10). The first absorbent pant article may for example be an elastic film laminate pant as described and shown in Figs. 1-7 and the second absorbent pant article may for example be a threaded pant as described and shown in Figs. 8-13. In each of Figures 14-16, figure a shows the inside and figure b shows the outside.

The absorbent articles 201, 301, 401 respectively comprise information tags 215, 315, 415 arranged on the inside of said waist portion, each time in substantially the same position. The material of the information tags is in a third colour (i.e. the tags have all the same colour) which is different from the first and second colours (of the first article, respectively the second and third articles) by a colour difference ∆E*ab more than 20. In this way, the information tags are clearly distinguishable by users and indicate which side of the article is the inside. If a user wants to reuse an absorbent article they will easily see which side is the inside, which might be difficult if the absorbent article is fully coloured. Fully coloured means that the whole article is coloured. That is, for example all outer and inner nonwoven sheets of a product are fully coloured including the topsheet.. That is, each sheet has a base colour which consist of a primary colour such as black, red, blue, violet, orange, yellow, green and indigo, as well as any other hue or mix thereof. These colours may also be mixed with white.. Sheets underneath the outer and inner nonwoven sheets may also be fully coloured and influence the colour of product, i.e. the colour of the outside and inside surface, That is, they have a base colour which consist of a primary colour such as black, red, blue, violet, orange, yellow, green and indigo, as well as any other hue or mix thereof. These colours may also be mixed with white. The sheets underneath the outer of inner non-woven material may also be white. Alternatively, the outer and inner nonwoven sheets may be thin white or transparent nonwovens. In order to get a fully coloured product the sheets under the outer and inner nonwoven sheets should be coloured and shine through the outer and inner thin white or transparent nonwoven sheets.

The information tags 215, 315, 415 may printed with symbols such as brand name, logotype, size indication, the wording "back" or "front", back/front indication, absorption level, artwork etc. The tag is fixedly attached on the inner wearer facing side of the chassis, preferably to the back portion 206, 306, 406 and/or to the waistband attached to the back portion. The tag provides a visual indicator for differentiating between the front and back portion of the article and thereby facilitate proper fitting of the article to the wearer. Alternatively the tag can be attached to the front portion 205, 305, 405 and/or to the waistband attached to the front portion to aid a young child to properly step into the article. Young children tend to look at the front of a pant article and align it with the stomach rather than searching for a back indicator. Adults, however, are often used to labels in ordinary underwear that are located at the back of the underwear. An adult would therefore likely associate a tag or label with the back portion of a pant. Since ordinary underwear usually is provided with a tag having printed thereon brand name, logotype, size, washing instructions etc. the provision of such as tag will make the absorbent article more underwear-like and enhance the appearance of the absorbent article.

The information tags 215, 315, 415 are shown to have a rectangular shape, but may of course have any optional shape. The size can vary but preferably it has a dimension in the transverse direction, x, of the article of at least 30 mm and up to 120 mm and in longitudinal direction, y, of at least 20 mm and up to 90 mm.

The information tags 215, 315, 415 are preferably made of a nonwoven material suited for printing on. Examples of suitable materials are spunbond, meltblown and laminates thereof, for example SMS materials (spunbond-meltblown-spunbond), embossed plastic film, laminates of plastic/film/nonwoven, textile material etc.

The information tags 215, 315, 415 can be fully attatched or party attatched. i.e. have an unattached portion. In order for the tag not to give skin irritations or be noticeable when the product is worn by a wearer, the bending rigidity of the unattached portion of the tag is a parameter of interest. Usual laundry tags in normal underwear are commonly accepted. The tag material should preferably have a Bending rigidity according to Kawabata of not more than 0.1 gf-cm$^2$/cm, preferably not more than 0.05 gf-cm$^2$/cm, in order to ensure that the unattached portion will not cause any skin irritation. This value is calculated as a mean value of the bending rigidity in MD and CD directions.

The attachment of the information tags 215, 315, 415 to the chassis may be made by adhesive, ultrasonic welding, thermal bonding or other ways, preferably by adhesive. In case the tag is applied under the waistband, it may be attached by thermal bonding or ultrasonic welding, in case thermal bonding or ultrasonic welding is used for attaching the waistband to the front/back portion. Combinations of adhesive and thermal bonding or ultrasonic welding may be used as well.

In order to provide a fixedly attachment of the information tags 215, 315, 415 which also withstands shear forces during use of the article, the shear force of the attachment should be sufficiently high as well as the peel force.

In the process for attaching the tags to the articles the attachment, for example adhesive, does not have to be applied all the way to at least one side edge of the tag, which simplifies the process and reduces the risk that adhesive ends up outside the tag area, where it may cause skin irritations.

In order to enhance the possibility to differentiate the information tags 215, 315, 415 from the surrounding portions of the chassis by the fingertips, the tags may have a surface structure that differs from the surrounding parts of the chassis.

In the embodiments shown in figs. 14-16, the articles are fully coloured, i.e. the inside and the outside of the product have the same colour. Preferably the inside as well as the outer fabrics have the same colour. Other materials/fabrics under the outer and inner fabrics may be coloured and may influence the colour of the absorbent article.

The following of the above mentioned fabrics are examples of inner fabrics of an elastic film laminate pant according to Figs. 1-7 and according to the present disclosure: inner layer(s) 21 or 36 of laminate 20, the inside of waist band 18, backsheet 25 (can also be an outer fabric), topsheet 26, cover strip 35. These are the materials or fabrics of which at least a part in use faces the user's skin. The inner fabrics are part of a plurality of fabrics which define the colours of the inside surface of the absorbent article.

The following of the above-mentioned fabrics are examples of outer fabrics of an elastic film laminate pant according to Figs. 1-7 and according to the present disclosure: outer layer(s) 22 or 38 of laminate 20, the outside of waist band 18, crotch panel material 24 and/or backsheet 25. These are the materials or fabrics of which at least a part in use faces the user's clothing. The outer fabrics are part of a plurality of fabrics which define the colour of the outside surfaces of the absorbent article.

The following of the above mentioned fabrics are examples of inner fabrics of threaded pant according to Figs. 8-13 and according to the present disclosure: topsheet 132 for the back and front section 4 and topsheet 132 for crotch section. These are the materials or fabrics of which at least a part in use faces the user's skin. The inner fabrics are part of a plurality of fabrics which define the colours of the inside surface of the absorbent article.

**The** following of the above mentioned fabrics are examples of outer fabrics of a threaded pant according to Figs. 8-13 and according to the present disclosure: backsheet 133 for front and back section 3, 4 and backsheet for crotch portion. These are the materials of which at least a part in use faces the user's clothing. The outer fabrics are part of a plurality of fabrics which define the colour of the outside surfaces of the absorbent article.

The colour of the inside and outside surfaces, or portions thereof, may result from the combination of all layers of the fabric, depending on e.g. the density of a non-woven fabric and the resulting transparency or opacity.

Figure 14a shows the inside surface of the pant with inside surface portions 205a, 206a of respectively the front 205 and back portions 206 of the chassis or waist portion of the first article 201. The connecting or crotch portion 219 comprises, on top, the liquid-permeable topsheet 216, which has an outer zone 213 surrounding the absorbent core, also referred to as a surrounding zone on the topsheet, and an inner or absorption zone 212 above the core 202. In embodiments, within the absorption zone 212, a central region 211 may be distinguishable by presence of an acquisition layer on top of the core. These are the portions of the inside surface which in use faces the user's skin. The inside surface portions comprise a plurality of fabrics/materials which define the colours of the inside surface of the absorbent article. The inside surface portions 205a, 206a of respectively the front and back portions 205, 206 and the topsheet with its different zones are examples of different portions of the inside surface.

Figure 14b shows the outside surface of the pant with outside portions 205b, 206b of respectively the front and back portions 205, 206 of the chassis or waist portion of the first article 201. The connecting or crotch portion 219 comprises on the outside a zone 214 which may be formed by the crotch panel material described above. These are the materials which in use faces the user's clothing. The outer fabrics are part of a plurality of fabrics which define the colour of the outside surfaces of the absorbent article. The outside surface portions 205a, 206a and zone 214 are examples of different portions of the outside surface.

In embodiments, at least the outside surface portions 214, 205b, 206b and the zone 213 on the liquid-permeable topsheet 216 (at least partly) surrounding the absorption zone 212 have, in the CIE L*a*b* colour space, measured as described below, L* values less than 80 and mutual colour differences ΔE*ab less than 10, i.e. these fabrics are coloured with substantially the same colour. Preferably, also the inside portions 205a, 205b have substantially this same colour.

The absorption zone 212 may have an L* value less than 90 and a colour difference ΔE*ab more than 5 with respect to zone 213 surrounding the absorption zone 212 and/or the outside portions 205b, 206b. The absorption zone 212 is thus also coloured for reasons of discreetness, but may have a noticeable colour difference with the surrounding zone and/or outside surface and can be reassured that the absorbent core is present. In embodiments, the indication of the absorption zone 212 may be provided by the absorbent core 202, or at least an upper layer thereof, being visible through the topsheet 216. This means that the colour difference of the absorption zone 212, which indicates its presence to the user, may be achieved by the visibility of the core or upper layer thereof, through the topsheet 216. In this way, the presence of the absorbent core can be indicated to the user without the need of providing print or other indications on the topsheet 416 itself. The visibility of the absorbent core 202, or at least an upper layer thereof, may for example be achieved by the core being made of a material which has a higher L* value (i.e. has a lighter colour) than the topsheet 216. The colour of the absorption zone 212 thus results from the combination of the colours of the absorbent core and the topsheet 416 (which is preferably per se entirely of the same colour/fabric) and may have a higher L* value (i.e. have a lighter colour) than said zone 213 surrounding the absorption zone, ΔL* between these zones being for example more than 5, preferably more than 10 and less than 35.

In embodiments, the material of the absorbent core 202 or upper layer thereof may have an L* value of more than 80, i.e. be of a light colour which may be clearly visible through the topsheet 216.

In embodiments according to the disclosure, all the inside and outside portions except in the absorption zone 212, may have L* values less than 80, preferably less than 60 (i.e. have relatively dark colours), and/or mutual colour differences ΔE*ab less than 10. It has been found that darker colours are preferred by users for reasons of discreetness and/or more underwear-like appearance.

The second article 301, shown in Figures 15a-b, is structurally the same as the first article, i.e. all the following shown elements are structurally the same as the respective elements of the first article: front portion 305 with inside and outside surface portions of the inside and outside surface 305a, 305b, back portion 306 with inside and outside surface portions of the inside and outside surface 306a, 306b, and crotch portion 319 comprising liquid-permeable topsheet 316 with outer zone 313, absorption zone 312 defined by the absorbent core 302 and central zone 311 defined by acquisition layer, and on the outside portion 314. A difference is that the inside and outside surface portions of the second article 301 have a darker colourthan the respective portions of the first article 201.

The third article 401, shown in Figures 16a-b, is structurally different from the second article shown in Figures 15a-b, in particular the third article 401 may be considered as intended for female users whereas the second article 301 may be considered as intended for male users. Structural differences shown are the shape or cut of the front portion 405 and the location of the acquisition layer 411 on the crotch portion 419. The other elements are structurally the same: back portion 406 with inside and outside portions of the inside and outside surface 406a, 406b, crotch portion 419 (except for the central zone / acquisition layer 411) with liquid-permeable topsheet 416 with outer zone 413, absorption zone 412 defined by the absorbent core 402 and on the outside portion 414. The visible elements of the third article 401, in particular outside surface portions 405b, 406b, 444 and inside surface portions 405a, 406a, 416, have the same colour as the respective inside and outside portions of the second article 301.

In embodiments, the articles 201, 301, 401 may be fully coloured, i.e. they have a base colour which consist of a primary colour such as black, red, blue, violet, orange, yellow, green and indigo, as well as any other hue or mix thereof. These colours may also be mixed with white. That is, the sheets of material, i.e. the plurality of fabrics which define the colour of the outside and inside of the pant article may be fully coloured, i.e. they have a base colour which consist of a primary colour such as black, red, blue, violet, orange, yellow, green and indigo, as well as any other hue or mix thereof. These colours may also be mixed with white. The colour can be added to the material either upon manufacture of it, for example by impregnating, or supplied afterwards by known print techniques. Examples of print technique can be flexography, ink-jet printers etc. The colouration may be made by impregnation of a colourant into a substrate. Colourants such as dyes, pigments, or combinations may be impregnated in the formation of substrates such as polymerics, resins, or nonwovens. For example, the colourant may be added to molten batch of polymer during film, fiber, or filament formation. All methods are well known in the art.

Colour measurement

Colour is determined within the L*a*b* colour space, as established by the Commission Internationale de l'Éclairage (CIE) in 1976. The colour space is divided into three axes. L* represents lightness and the axis extends from 0 (black) to 100 (white). The axis a* goes from green to red, where positive values indicate more saturated red, and negative values more saturated green. The b*-axis goes from blue to yellow, where positive values represent more saturated yellow and negative values more saturated blue. This colour space is well known in industry and is generally referred to as CIE L*a*b* or CIELAB (1976).

A suitable spectrophotometer is available from Konica-Minolta, under the designation CM-5 (equivalent apparatuses can also be used). The apparatus illuminates the sample diffusely and detects the light at 8 degrees to the normal line (a geometry named di:8°, de:8°). For this purpose, the instrument is set to SCE (specular component excluded). Standard Illuminant D65 is utilized, and the viewing angle is set to 10°. Normally the diameter of the measured area should be 30 mm. However if the relevant sample areas are smaller (having a diameter less than 30 mm) then smaller spectrophotometer apertures are utilized (as large as possible without transgressing the relevant colour area).

A difference between two colours in the colour space CIE L*a*b* is characterised by a Delta E-value ($\Delta E^*ab$). The differences between the colours on the three respective axes are squared in this, following which the differences are summed and the root derived from the sum:

$$\Delta E^*_{ab} = \sqrt{(L^*_2 - L^*_1)^2 + (a^*_2 - a^*_1)^2 + (b^*_2 - b^*_1)^2}$$

The following measurement procedure may be used. The pant part with the portion to be measured is placed over the recording aperture of the instrument. In particular, the sample is placed flat and smooth over the spectrophotometer aperture. Any elastic elements should remain in their contracted or non-stretched state. A white ceramic tile (available from Konica-Minolta) is placed on (above) the sample, as a standard backing. The ceramic tile has values L* 96.0, a* 0.1 and b* 2,85. The recording aperture of the instrument is placed against the sample/products. The measurement can then begin. Since the absorbent articles are in the form of pants, the sides, of which the front section and back section have been joined in a seam may be torn/clipped. However, the measurements may also be performed on a product where the reams not have been torn/clipped.

Examples

The above procedure was used for the examples mentioned below.

The absorbent articles are in the form of pants, the sides, of which the front section and back section are joined in a seam. The measurements were performed on products which the seams were not torn/clipped.

**The** portion of the absorbent article which was to be measured was placed over the (normally 30 mm diameter) aperture, and then a white ceramic tile on top. The portion is placed flat and smooth over the spectrophotometer aperture. Any elastic elements should remain in their contracted or non-stretched state. A white ceramic tile (available from Konica-Minolta) is placed on (above) the sample, as a standard backing. The ceramic tile has values L* 96.0, a* 0.1 and b* 2,85. The white tile mimics the user's pale skin when looking at the diaper from the outside The recording aperture of the instrument is placed against the sample/products. The measurement can then begin. In the examples below the tile will not shine through so much, but for a very thin chassis or a thin nonwoven laminate it can make the L-value higher

Example 1: Black threaded pant article similar construction as the pant described in connection with Figs. 8 -13.

The waist panel, is formed of a laminate consisting of two inelastic black nonwoven layers where elastic threads have been placed between. The elastic threads are laid crosswise in the transverse direction of the article. Examples of elastic threads are sold under the LYCRA trademark. The two nonwoven layers are connected to one another by two methods, construction adhesive coated on one of the non-woven and elastic adhesive coated on the elastic threads between the nonwoven layers. The two nonwoven layers are formed from hydrophobic spunbond and have a weight per unit area of 18 gsm (Gram per Square Meter) respectively. In this example a black spunbond nonwoven 18gsm from Avinti was used,

The backsheet under the core comprises the same non-woven as for the waist panel and it is attached to a coloured plastic film of polyethylene with a weight per unit area of 15gsm for black product (non breathable film) The film has the following value: L*= 26,3, a*: -0.1 and b*=-0,3. The film was measured as a single ply, with a white ceramic tile as background (the tile has values L* 96.0, a* 0.1 and b* 2,85). The same tile is used as background in all of our measurements, but on materials as thin as a single ply backsheet film it influences a bit more than on more massive and opaque objects.

The topsheet over the core and an area outside the core is liquid-permeable and is of a spunbonded, 16 gsm hydrophilic material. For this threaded product a spunbond Non woven 16gsm from Fitesa was used.

The product had a white absorbent core underneath the colored topsheet. The following values was measured on the different portions.

| Example 1 | L* | a* | b* |
|---|---|---|---|
| Panel (outside, portions 45b, 46b) | 18.0 | 0.63 | 1.87 |
| Backsheet under core (zone 44) | 22.9 | 0.63 | 1.72 |
| Topsheet over core (zone 42) | 35.1 | 0.86 | 4.63 |
| Topsheet area outside of the core (surrounding zone 43) | 18,5 | 0,46 | 1,47 |
| $\Delta$E*ab between Panel and Backsheet under core: 4.95 | | | |
| $\Delta$E*ab between Topsheet over core and Backsheet under core: 12.52 | | | |
| $\Delta$E*ab between Topsheet over core and Topsheet area outside of the core; 16,19 | | | |

Example 2: Black elastic film laminate pants - similar construction as the pant described in connection with Figs. 1 -7

The waist panel is formed of a laminate consisting of two inelastic black nonwoven layers where a black elastic film (35 gsm from Tredegar) has been placed between them.

The two nonwoven layers are formed from hydrophobic spunbond and have a weight per unit area of 16 g/m<2> respectively. For this product the following material has been used: S-Tex Noir Cal 12% from Berry, Fiberweb France SAS. The two nonwoven layers are connected to the film by ultrasonic welding.

The backsheet under the core comprises the same non-woven as for the waist panel has. The Non-woven is attached to a black colored plastic film of polyethylene with a weight per unit area of 15 gsm. The film has the following value: L*= 26,3, a*: -0.1 and b*=-0,3. The film was measured as a single ply, with a white ceramic tile as background (the tile has values L* 96.0, a* 0.1 and b* 2,85). The same tile is used as background in all of our measurements, but on materials as thin as a single ply backsheet film it influences a bit more than on more massive and opaque objects.

The topsheet is liquid-permeable and is formed in this case of the same non-woven as the waist panel, i.e. spunbonded, 15,8 gsm S-Tex Noir Cal 12% from Berry, Fiberweb France SAS. The topsheet has been treated to become hydrophilic.

The product had a white absorbent core underneath the colored topsheet. The following values was measured on the different portions.

| Example 2 | L* | a* | b* |
|---|---|---|---|
| Panel (outside, zones 45b, 46b) | 13.6 | 0.42 | 0.88 |
| Backsheet under core (zone 44) | 19.5 | 0.33 | 0.82 |
| Topsheet over core (zone 42) | 41.5 | 0.37 | 3.86 |

(continued)

| Example 2 | L* | a* | b* |
|---|---|---|---|
| Topsheet area outside of the core (surrounding zone 43) | 19,0 | 0,26 | 0,9 |
| ΔE*ab between Panel and Backsheet under core: 5.88 | | | |
| ΔE*ab between Topsheet over core and Backsheet under core: 12.52 | | | |
| ΔE*ab between Topsheet over core and Topsheet area outside of the core: 22,69 | | | |

Example 3: Burgundy elastic film laminate pants - similar construction as the pant described in connection with Figs. 1 -7.

The waist panel, is formed of a laminate consisting of two inelastic burgundy coloired nonwoven layers where a black elastic film (35 gsm from Tredegar) has been placed between them.

The two nonwoven layers are formed from hydrophobic spunbond and have a weight per unit area of 16 g/m<2> respectively. For this product the following material has been used:
S-Tex Noir Cal 12% from Berry, Fiberweb France SAS. The two nonwoven layers are connected to the film by ultrasonic welding.

The backsheet under the absorbent core comprises the same non-woven material as for the waist panel. The Non-woven is attached to a black coloured plastic film of polyethylene with a weight per unit area of 15 gsm. The film has the following value: L*=42.2, a*= -25,5 and b*=--4,7. The film was measured as a single ply, with a white ceramic tile as background (the tile has values L* 96.0, a* 0.1 and b* 2,85). The same tile is used as background in all of our measurements, but on materials as thin as a single ply backsheet film it influences a bit more than on more massive and opaque objects.

The topsheet is liquid-permeable and is formed in this case of the same NW as the waist panel, i.e. spunbonded, 15,8 g/m<2>, S-Tex Noir Cal 12% from Berry, Fiberweb France SAS. The topsheet has been treated to become hydrophilic.

The product had a white core underneath the colored topsheet. The following values was measured on the different portions.

| Example 3 | L* | a* | b* |
|---|---|---|---|
| Panel (outside, zones 45b, 46b) | 21.7 | 14.1 | -3.29 |
| Backsheet under core (zone 44) | 28.1 | 16.24 | -3.61 |
| Topsheet over core (zone 42) | 40.0 | 20.37 | -3.14 |
| Topsheet area outside of the core (surrounding zone 43) | 25,6 | 15,14 | -4,01 |
| ΔE*ab between Panel and Backsheet under core: 6.69 | | | |
| ΔE*ab between Topsheet over core and Backsheet under core: 12.64 | | | |
| ΔE*ab between Topsheet over core and Topsheet area outside of the core: 15,37 | | | |

Example 4: Pink-Beige Threaded Pants - similar construction as the pant described in connection with Figs. 8-13

The waist panel, is formed of a laminate consisting of two inelastic pink-beige coloured nonwoven layers where elastic threads have been placed between them. The elastic threads are laid crosswise in the transverse direction of the article. Examples of elastic threads are sold under the LYCRA or SPANDEX trademark. The two nonwoven layers are connected to one another by two methods, construction adhesive coated on one of the non-woven layers and elastic adhesive coated on the elastic threads between the nonwoven layers. The two nonwoven layers are formed from hydrophobic spunbond and have a weight per unit area of 18 gsm respectively. In this example a spunbond nonwoven 18gsm from Avinti was used.

The backsheet under the core comprises the same NW as for the waist panel and it is attached to a colored plastic film of polyethylene with a weight per unit area of 20gsm (pink, breathable film). The film has the following value: L*= 83,5, a*= 8,9 and b*=-8,6. The film was measured as a single ply, with a white ceramic tile as background (the tile has values L* 96.0, a* 0.1 and b* 2,85). The same tile is used as background in all of our measurements, but on materials as thin as a single ply backsheet film it influences a bit more than on more massive and opaque objects.

The topsheet over the core and an area outside the core is liquid-permeable and is of a spunbonded, 16 gsm hydrophilic material. For this threaded product a spunbond non-woven 16gsm from Fitesa was used.

The product had a white absorbent core underneath the colored topsheet. The following values was measured on the different portions.

| Example 4 | L* | a* | b* |
|---|---|---|---|
| Panel (outside, portions 45b, 46b) | 70.2 | 11.9 | 13.26 |
| Backsheet under core (zone 44) | 78.9 | 10.15 | 12.07 |
| Topsheet over core (zone 42) | 86.8 | 5.45 | 12.47 |
| Topsheet area outside of the core (surrounding zone 43) | 73,1 | 9,14 | 10,21 |
| $\Delta$E*ab between Panel and Backsheet under core: 8.95 | | | |
| $\Delta$E*ab between Topsheet over core and Backsheet under core: 9.19 | | | |
| $\Delta$E*ab between Topsheet over core and Topsheet area outside of the core: 14,41 | | | |

These examples show that different kind of absorbent pant products having different kind of colours. The products are coloured both on the inside surface and on the outside surface. The absorption zone is coloured for reasons of discreetness, but which has a noticeable colour difference with the surrounding zone and the outside surface. However, the topsheet may be fully coloured so that the absorption zone is not noticeable from the surrounding zone and the outside surface. In both cases an information tag in a third colour which is different from the first and second colours of the products by a colour difference $\Delta$E*ab more than 20; will clearly indicate which side ins the inside, even for light coloured products, such as pink beige products.

The portion: "Panel (outside, portions 45b, 46b)" in the tables above can also be considered being the inside portions of the panels since the same non-woven is placed on each side of the elastic film or threads, hence the waist panel will show the same colour both on the inside of the product and on the outside of the products.

If a separate piece of material for forming standing gather or leg elastic is attached to the topsheet in the area of the surrounding zone the measurements should be made to a portion where the topsheet is provided, that is shown. This can for example be in the front or the back of the pant article, since usually standing gathers and leg elastics are only attached on the sides of the absorbent in the crotch portion. The separate piece of material for forming standing gather or leg elastic is advantageously also coloured in the same colour as the other portions for example the outside surface, i.e. having a colour differences $\Delta$E*ab less than 10.

All the above examples are absorbent products which are fully coloured, i.e. they have a base colour which they have a base colour which consist of a primary colour such as black, red, blue, violet, orange, yellow, green and indigo, as well as any other hue or mix. The colours may also be mixed with white. The sheets of material, i.e. both the non-woven and plastic films are fully coloured i.e. they also have a base colour which consist of a primary colour such as black, red, blue, violet, orange, yellow, green and indigo, as well as any other hue or mix thereof, The colours may also be mixed with white.

A fully coloured pant may additional have a discrete graphic, such as flowers on the outside of the product. In this case the background colour and not the graphic shall be measured and compared.

## Claims

1. An array comprising at least one first disposable pant article and at least one second disposable pant article, wherein

   each first disposable pant article has a first structure, comprising a waist portion which in use surrounds a user's waist and a crotch portion connecting front and back portions of the waist portion, the crotch portion comprising an absorbent core for absorbing body exudates and a liquid-permeable topsheet covering the absorbent core, wherein the article has an inside surface which in use faces the user's skin and an outside surface which is opposite the inside surface and in use faces the user's clothing; wherein the article is composed of a plurality of fabrics which define the colours of the inside and outside surfaces of the article, the liquid-permeable topsheet being one of the fabrics; wherein an absorption zone is defined as the part of the inside surface, on the topsheet, under which the absorbent core is located, and wherein a surrounding zone is defined as the part of the inside surface, on the topsheet, adjacent to and at least partly surrounding the absorption zone; and
   each second disposable pant article has a second structure, comprising a waist portion which in use surrounds a user's waist and a crotch portion connecting front and back portions of the waist portion, the crotch portion comprising an absorbent core for absorbing body exudates and a liquid-permeable topsheet covering the absorbent core while indicating an absorption zone where the absorbent core is located, wherein the article has an inside surface which in use faces the user's skin and an outside surface which is opposite the inside surface and in use faces the user's clothing; wherein the article is composed of a plurality of fabrics which define the colours of the inside and outside surfaces of the article, the liquid-permeable topsheet being one of the fabrics; wherein an absorption zone is defined as the part of the inside surface, on the topsheet, under which the absorbent core is located, and wherein a surrounding zone is defined as the part of the inside surface, on the topsheet, adjacent to and at least partly surrounding the absorption zone; wherein

for each first disposable pant article, at least the outside surface and said surrounding zone have substantially the same first colour with, in the CIE L\*a\*b\* colour space, L\* values less than 80 and mutual colour differences ∆E\*ab less than 10;

for each second disposable pant article, at least the outside surface and said surrounding zone have substantially the same second colour with L\* values less than 80 and mutual colour differences ∆E\*ab less than 10;

said outside surface of said first and/or second disposable pant article comprises outside surface portions, together forming said outside surface, and said inside surface of said first and/or second disposable pant article comprises inside surface portions, together forming said inside surface, and all the outside and inside surface portions of said first article, possibly except in said absorption zone, have L\* values less than 80 and mutual colour differences ∆E\*ab less than 10 and/or all the outside and inside surface portions of said second article, possibly except in said absorption zone, have L\* values less than 80 and mutual colour differences ∆E\*ab less than 10;

each of the first and second disposable pant articles comprises an information tag arranged on the inside of said waist portion and providing a visual indicator for differentiating between the front and back portions of the respective article, the material of the information tag being in a third colour which is different from the first and second colours by a colour difference ∆E\*ab more than 20; and

the first structure is structurally different from the second structure and/or the first colour is different from the second colour by a colour difference ∆E\*ab more than 10.

2. The array according to claim 1, wherein all the inside and outside surface portions of said first article except in said absorption zone, have L\* values less than 80 and mutual colour differences ∆E\*ab less than 10 and/or all the inside and outside surface portions of said second article except in said absorption zone, have L\* values less than 80 and mutual colour differences ∆E\*ab less than 10.

3. The array according to claim 1, wherein all the inside and outside surface portions of said first article except in said absorption zone, have L\* values less than 60 and mutual colour differences ∆E\*ab less than 10 and/or all the inside and outside surface portions of said second article except in said absorption zone, have L\* values less than 60 and mutual colour differences ∆E\*ab less than 10.

4. The array according to claim 1, wherein all the inside and outside surface portions of said first article have L\* values less than 80 and mutual colour differences ∆E\*ab less than 10 and/or all the inside and outside surface portions of the second disposable pant article have L\* values less than 80 and mutual colour differences ∆E\*ab less than 10.

5. The array according to claim 1, wherein all the inside and outside surface portions of said first article have L\* values less than 60 and mutual colour differences ∆E\*ab less than 10 and/or all the inside and outside surface portions of the second disposable pant article have L\* values less than 60 and mutual colour differences ∆E\*ab less than 10.

6. The array according to claim 1, wherein the absorption zone of said first and/or second disposable pant article has an L\* value less than 90 and a colour difference ∆E\*ab more than 5 with respect to said surrounding zone and/or said outside surface.

7. The array according to any one of claims claim 1-6, wherein the structural difference is chosen from: that the first article is optimized for male users whereas the second article is optimized for female users; that the first and second articles have different sizes; that the first article is a threaded pant article comprising elastic threads in the waist portion, whereas the second article is not such a threaded pant article; that the first article is a layered pant article comprising an elastic laminate in the waist portion, whereas the second article is not such a layered pant article.

8. The array according to any one of the preceding claims, wherein the first and second colours are substantially the same.

9. The array according to any one of claims 1-7 wherein the first and second colours are different by a colour difference ΔE*ab more than 10.

10. The array according to any one of the preceding claims, wherein the first and second colours have L* values less than 60.

11. The array according to any one of the preceding claims, wherein for each of said first and second articles, the information tag is arranged on the back portion of the waist portion.

12. The array according to any one of the preceding claims, wherein for each of said first and second articles, the absorbent core is visible through the liquid-permeable topsheet.

13. The array according to any one of the preceding claims, wherein said information tags are printed or embossed.

14. The array according to any one of the preceding claims, wherein for each of said first and second articles, lot codes or lot numbers or production codes are located on said information tags.

15. A method for producing an array of disposable pant articles, comprising the steps of:

producing a plurality of first articles having a first structure, comprising a waist portion which in use surrounds a user's waist and a crotch portion connecting front and back portions of the waist portion, the crotch portion comprising an absorbent core for absorbing body exudates and a liquid-permeable topsheet covering the absorbent core wherein the article has an inside surface which in use faces the user's skin and an outside surface which is opposite the inside surface and in use faces the user's clothing; wherein the article is composed of a plurality of fabrics which define the colours of the inside and outside surfaces of the article, the liquid-permeable topsheet being one of the fabrics; wherein an absorption zone is defined as the part of the inside surface, on the topsheet, under which the absorbent core is located, and wherein a surrounding zone is defined as the part of the inside surface, on the topsheet, adjacent to and at least partly surrounding the absorption zone; wherein for each first disposable pant article, at least the outside surface and said surrounding zone have substantially the same first colour with, in the CIE L*a*b* colour space, L* values less than 80 and mutual colour differences ΔE*ab less than 10;
producing a plurality of second articles having a first structure, comprising a waist portion which in use surrounds a user's waist and a crotch portion connecting front and back portions of the waist portion, the crotch portion comprising an absorbent core for absorbing body exudates and a liquid-permeable topsheet covering the absorbent core wherein the article has an inside surface which in use faces the user's skin and an outside surface which is opposite the inside surface and in use faces the user's clothing; wherein the article is composed of a plurality of fabrics which define the colours of the inside and outside surfaces of the article, the liquid-permeable topsheet being one of the fabrics; wherein an absorption zone is defined as the part of the inside surface, on the topsheet, under which the absorbent core is located, and wherein a surrounding zone is defined as the part of the inside surface, on the topsheet, adjacent to and at least partly surrounding the absorption zone; wherein for each second disposable pant article, at least the outside surface and said surrounding zone have substantially the same second colour with L* values less than 80 and mutual colour differences ΔE*ab less than 10;
wherein said outside surface of said first and/or second disposable pant article comprises outside surface portions, together forming said outside surface, and said inside surface of said first and/or second disposable pant article comprises inside surface portions, together forming said inside surface, and all the outside and inside surface portions of said first article, possibly except in said absorption zone, have L* values less than 80 and mutual colour differences ΔE*ab less than 10 and/or all the outside and inside surface portions of said second article, possibly except in said absorption zone, have L* values less than 80 and mutual colour differences ΔE*ab less than 10;
wherein for each of said first and second disposable pant articles the first structure is structurally different from the second structure and/or the first colour is different from the second colour by a colour difference ΔE*ab more than 10; and
attaching information tags on the inside of said waist portions of each of the first and second articles, thereby providing a visual indicator for differentiating between the front and back portions of the respective article; the material of the information tag being in a third colour which is different from the first and second colours by a colour difference ΔE*ab more than 20.

**Patentansprüche**

1. Anordnung, die mindestens einen ersten Einweg-Hosenartikel und mindestens einen zweiten Einweg-Hosenartikel umfasst, wobei

jeder erste Einweg-Hosenartikel eine erste Struktur aufweist, die einen Taillenteil, der im Gebrauch die Taille eines Benutzers umgibt, und einen Schrittteil umfasst, der den vorderen und hinteren Teil des Taillenteils verbindet, wobei der Schrittteil einen absorbierenden Kern zum Absorbieren von Körperausscheidungen und eine flüssigkeitsdurchlässige Oberschicht umfasst, die den absorbierenden Kern bedeckt, wobei der Artikel eine Innenfläche, die im Gebrauch der Haut des Benutzers zugewandt ist, und eine Außenfläche aufweist, die der Innenfläche gegenüberliegt und im Gebrauch der Bekleidung des Benutzers zugewandt ist; wobei der Artikel aus einer Vielzahl von Stoffen besteht, die die Farben der Innen- und Außenfläche des Artikels definieren, wobei die flüssigkeitsdurchlässige Oberschicht einer der Stoffe ist; wobei ein Absorptionsbereich als der Teil der Innenfläche auf der Oberschicht definiert ist, unter dem der absorbierende Kern angeordnet ist, und wobei ein Umgebungsbereich als der Teil der Innenfläche auf der Oberschicht definiert ist, der an den Absorptionsbereich angrenzt und diesen zumindest teilweise umgibt; und

jeder zweite Einweg-Hosenartikel eine zweite Struktur aufweist, die einen Taillenteil, der im Gebrauch die Taille eines Benutzers umgibt, und einen Schrittteil umfasst, der den vorderen und hinteren Teil des Taillenteils verbindet, wobei der Schrittteil einen absorbierenden Kern zum Absorbieren von Körperausscheidungen und eine flüssigkeitsdurchlässige Oberschicht umfasst, die den absorbierenden Kern bedeckt und gleichzeitig einen Absorptionsbereich anzeigt, in dem der absorbierende Kern angeordnet ist, wobei der Artikel eine Innenfläche, die im Gebrauch der Haut des Benutzers zugewandt ist, und eine Außenfläche aufweist, die der Innenfläche gegenüberliegt und im Gebrauch der Bekleidung des Benutzers zugewandt ist; wobei der Artikel aus einer Vielzahl von Stoffen besteht, die die Farben der Innen- und Außenfläche des Artikels definieren, wobei die flüssigkeitsdurchlässige Oberschicht einer der Stoffe ist; wobei ein Absorptionsbereich als der Teil der Innenfläche auf der Oberschicht definiert ist, unter dem der absorbierende Kern angeordnet ist, und wobei ein Umgebungsbereich als der Teil der Innenfläche auf der Oberschicht definiert ist, der an den Absorptionsbereich angrenzt und diesen zumindest teilweise umgibt; wobei

bei jedem ersten Einweg-Hosenartikel zumindest die Außenfläche und der besagte Umgebungsbereich im Wesentlichen dieselbe erste Farbe in dem CIE-L*a*b*-Farbraum mit L*-Werten von weniger als 80 und gegenseitigen Farbunterschieden $\Delta E^*ab$ von weniger als 10 aufweisen;

bei jedem zweiten Einweg-Hosenartikel zumindest die Außenfläche und der besagte Umgebungsbereich im Wesentlichen dieselbe zweite Farbe mit L*-Werten von weniger als 80 und gegenseitigen Farbenunterschieden $\Delta E^*ab$ von weniger als 10 aufweisen;

die besagte Außenfläche des besagten ersten und/oder zweiten Einweg-Hosenartikels Außenflächenteile umfasst, die zusammen die besagte Außenfläche bilden, und die besagte Innenfläche des besagten ersten und/oder zweiten Einweg-Hosenartikels Innenflächenteile umfasst, die zusammen die besagte Innenfläche bilden, und alle Außen- und Innenflächenteile des besagten ersten Artikels, möglicherweise außer in dem besagten Absorptionsbereich, L*-Werte von weniger als 80 und gegenseitige Farbunterschiede $\Delta E^*ab$ von weniger als 10 aufweisen und/oder alle Außen- und Innenflächenteile des besagten zweiten Artikels, möglicherweise außer in dem besagten Absorptionsbereich, L*-Werte von weniger als 80 und gegenseitige Farbunterschiede $\Delta E^*ab$ von weniger als 10 aufweisen;

jeder des ersten und zweiten Einweg-Hosenartikels ein Informationsetikett umfasst, das an der Innenseite des besagten Taillenteils angeordnet ist und einen visuellen Indikator zum Differenzieren zwischen dem vorderen und hinteren Teil des betreffenden Artikels bereitstellt, wobei das Material des Informationsetiketts eine dritte Farbe aufweist, die sich von der ersten und zweiten Farbe um einen Farbunterschied $\Delta E^*ab$ von mehr als 20 unterscheidet; und

wobei sich die erste Struktur von der zweiten Struktur strukturell unterscheidet und/oder wobei sich die erste Farbe von der zweiten Farbe um einen Farbunterschied $\Delta E^*ab$ von mehr als 10 unterscheidet.

2. Anordnung nach Anspruch 1, wobei alle Innen- und Außenflächenteile des besagten ersten Artikels, außer in dem besagten Absorptionsbereich, L*-Werte von weniger als 80 und gegenseitige Farbunterschiede $\Delta E^*ab$ von weniger als 10 aufweisen und/oder alle Innen- und Außenflächenteile des besagten zweiten Artikels, außer in dem besagten Absorptionsbereich, L*-Werte von weniger als 80 und gegenseitige Farbunterschiede $\Delta E^*ab$ von weniger als 10 aufweisen.

3. Anordnung nach Anspruch 1, wobei alle Innen- und Außenflächenteile des besagten ersten Artikels, außer in dem besagten Absorptionsbereich, L*-Werte von weniger als 60 und gegenseitige Farbunterschiede $\Delta E^*ab$ von weniger

als 10 aufweisen und/oder alle Innen- und Außenflächenteile des besagten zweiten Artikels, außer in dem besagten Absorptionsbereich, L\*-Werte von weniger als 60 und gegenseitige Farbunterschiede ∆E\*ab von weniger als 10 aufweisen.

4. Anordnung nach Anspruch 1, wobei alle Innen- und Außenflächenteile des besagten ersten Artikels L\*-Werte von weniger als 80 und gegenseitige Farbunterschiede ∆E\*ab von weniger als 10 aufweisen und/oder alle Innen- und Außenflächenteile des zweiten Einweg-Hosenartikels L\*-Werte von weniger als 80 und gegenseitige Farbunterschiede ∆E\*ab von weniger als 10 aufweisen.

5. Anordnung nach Anspruch 1, wobei alle Innen- und Außenflächenteile des besagten ersten Artikels L\*-Werte von weniger als 60 und gegenseitige Farbunterschiede ∆E\*ab von weniger als 10 aufweisen und/oder alle Innen- und Außenflächenteile des zweiten Einweg-Hosenartikels L\*-Werte von weniger als 60 und gegenseitige Farbunterschiede ∆E\*ab von weniger als 10 aufweisen.

6. Anordnung nach Anspruch 1, wobei der Absorptionsbereich des besagten ersten und/oder zweiten Einweg-Hosenartikels einen L\*-Wert von weniger als 90 und einen Farbunterschied ∆E\*ab von mehr als 5 in Bezug auf den besagten Umgebungsbereich und/oder die besagte Außenfläche aufweist.

7. Anordnung nach einem der Ansprüche 1-6, wobei die strukturelle Differenz aus Folgendem ausgewählt ist: dass der erste Artikel für männliche Benutzer optimiert ist, während der zweite Artikel für weibliche Benutzer optimiert ist; dass der erste und zweite Artikel unterschiedliche Größen haben; dass der erste Artikel ein mit Fäden versehener Hosenartikel ist, der elastische Fäden in dem Taillenteil umfasst, während der zweite Artikel kein solcher mit Fäden versehener Hosenartikel ist; dass der erste Artikel ein geschichteter Hosenartikel ist, der ein elastisches Laminat in dem Taillenteil umfasst, während der zweite Artikel kein solcher geschichteter Hosenartikel ist.

8. Anordnung nach einem der vorhergehenden Ansprüche, wobei die erste und zweite Farbe im Wesentlichen gleich sind.

9. Anordnung nach einem der Ansprüche 1-7, wobei sich die erste und zweite Farbe um einen Farbunterschied ∆E\*ab von mehr als 10 unterscheiden.

10. Anordnung nach einem der vorhergehenden Ansprüche, wobei die erste und zweite Farbe L\*-Werte von weniger als 60 aufweisen.

11. Anordnung nach einem der vorhergehenden Ansprüche, wobei bei jedem des besagten ersten und zweiten Artikels das Informationsetikett an dem hinteren Teil des Taillenteils angeordnet ist.

12. Anordnung nach einem der vorhergehenden Ansprüche, wobei bei jedem des besagten ersten und zweiten Artikels der absorbierende Kern durch die flüssigkeitsdurchlässige Oberschicht sichtbar ist.

13. Anordnung nach einem der vorhergehenden Ansprüche, wobei die besagten Informationsetiketten gedruckt oder geprägt sind.

14. Anordnung nach einem der vorhergehenden Ansprüche, wobei sich bei jedem des besagten ersten und zweiten Artikels Lot-Codes oder Lot-Zahlen oder Produktions-Codes auf den besagten Informationsetiketten befinden.

15. Verfahren zum Herstellen einer Anordnung von Einweg-Hosenartikeln, das die folgenden Schritte umfasst:

Herstellen einer Vielzahl von ersten Artikeln mit einer ersten Struktur, die einen Taillenteil, der im Gebrauch die Taille eines Benutzers umgibt, und einen Schrittteil umfasst, der den vorderen und hinteren Teil des Taillenteils verbindet, wobei der Schrittteil einen absorbierenden Kern zum Absorbieren von Körperausscheidungen und eine flüssigkeitsdurchlässige Oberschicht umfasst, die den absorbierenden Kern bedeckt, wobei der Artikel eine Innenfläche, die im Gebrauch der Haut des Benutzers zugewandt ist, und eine Außenfläche aufweist, die der Innenfläche gegenüberliegt und im Gebrauch der Bekleidung des Benutzers zugewandt ist; wobei der Artikel aus einer Vielzahl von Stoffen besteht, die die Farben der Innen- und Außenfläche des Artikels definieren, wobei die flüssigkeitsdurchlässige Oberschicht einer der Stoffe ist; wobei ein Absorptionsbereich als der Teil der Innenfläche auf der Oberschicht definiert ist, unter dem der absorbierende Kern angeordnet ist, und wobei ein Umgebungsbereich als der Teil der Innenfläche auf der Oberschicht definiert ist, der an den Absorptionsbereich angrenzt und diesen zumindest teilweise umgibt; wobei bei jedem ersten Einweg-Hosenartikel zumindest die Außenfläche und der besagte Umgebungsbereich im Wesentlichen dieselbe erste Farbe in dem CIE-L*a*b*-Farbraum mit L*-Werten von weniger als 80 und gegenseitigen Farbunterschieden ∆E*ab von weniger als 10 aufweisen;

Herstellen einer Vielzahl von zweiten Artikeln mit einer ersten Struktur, die einen Taillenteil, der im Gebrauch die Taille eines Benutzers umgibt, und einen Schrittteil umfasst, der den vorderen und hinteren Teil des Taillenteils verbindet, wobei der Schrittteil einen absorbierenden Kern zum Absorbieren von Körperausscheidungen und eine flüssigkeitsdurchlässige Oberschicht umfasst, die den absorbierenden Kern bedeckt, wobei der Artikel eine Innenfläche, die im Gebrauch der Haut des Benutzers zugewandt ist, und eine Außenfläche aufweist, die der Innenfläche gegenüberliegt und im Gebrauch der Bekleidung des Benutzers zugewandt ist; wobei der Artikel aus einer Vielzahl von Stoffen besteht, die die Farben der Innen- und Außenfläche des Artikels definieren, wobei die flüssigkeitsdurchlässige Oberschicht einer der Stoffe ist; wobei ein Absorptionsbereich als der Teil der Innenfläche auf der Oberschicht definiert ist, unter dem der absorbierende Kern angeordnet ist, und wobei ein Umgebungsbereich als der Teil der Innenfläche auf der Oberschicht definiert ist, der an den Absorptionsbereich angrenzt und diesen zumindest teilweise umgibt; wobei bei jedem zweiten Einweg-Hosenartikel zumindest die Außenfläche und der besagte Umgebungsbereich im Wesentlichen dieselbe zweite Farbe mit L-*-Werten von weniger als 80 und gegenseitigen Farbunterschieden ∆E*ab von weniger als 10 aufweisen;

wobei die besagte Außenfläche des besagten ersten und/oder zweiten Einweg-Hosenartikels Außenflächenteile umfasst, die zusammen die besagte Außenfläche bilden, und die besagte Innenfläche des besagten ersten und/oder zweiten Einweg-Hosenartikels Innenflächenteile umfasst, die zusammen die besagte Innenfläche bilden, und alle Außen- und Innenflächenteile des besagten ersten Artikels, möglicherweise außer in dem besagten Absorptionsbereich, L*-Werte von weniger als 80 und gegenseitige Farbunterschiede ∆E*ab von weniger als 10 aufweisen und/oder alle Außen- und Innenflächenteile des besagten zweiten Artikels, möglicherweise außer in dem besagten Absorptionsbereich, L*-Werte von weniger als 80 und gegenseitige Farbunterschiede ∆E*ab von weniger als 10 aufweisen;

wobei sich bei jedem des besagten ersten und zweiten Einweg-Hosenartikels die erste Struktur von der zweiten Struktur strukturell unterscheidet und/oder wobei sich die erste Farbe von der zweiten Farbe um einen Farbunterschied ∆E*ab von mehr als 10 unterscheidet; und

Anbringen von Informationsetiketten an der Innenseite der besagten Taillenteile von jedem des ersten und zweiten Artikels, wodurch ein visueller Indikator zum Differenzieren zwischen dem vorderen und hinteren Teil des betreffenden Artikels bereitgestellt wird; wobei das Material des Informationsetiketts eine dritte Farbe aufweist, die sich von der ersten und zweiten Farbe um einen Farbunterschied ∆E*ab von mehr als 20 unterscheidet.

## Revendications

1. Réseau comprenant au moins un premier article de culotte jetable et au moins un deuxième article de culotte jetable, où

chaque premier article de culotte jetable a une première structure, comprenant une portion de taille qui, lors de l'utilisation, entoure la taille d'un utilisateur et une portion d'entrejambe reliant les portions avant et arrière de la portion de taille, la portion d'entrejambe comprenant une âme absorbante pour absorber des exsudats corporels et une feuille supérieure perméable aux liquides recouvrant l'âme absorbante, où l'article a une surface intérieure qui, lors de l'utilisation, fait face à la peau de l'utilisateur et une surface extérieure qui est opposée à la surface intérieure et, lors de l'utilisation, fait face aux vêtements de l'utilisateur ; où l'article est composé d'une pluralité de tissus qui définissent les couleurs des surfaces intérieures et extérieures de l'article, la feuille supérieure perméable aux liquides étant l'un des tissus ; où une zone d'absorption est définie comme la partie de la surface intérieure, sur la feuille supérieure, sous laquelle est située l'âme absorbante, et où une zone environnante est définie comme la partie de la surface intérieure, sur la feuille supérieure, adjacente à et au moins entourant en partie la zone d'absorption; et

chaque deuxième article de culotte jetable a une deuxième structure, comprenant une portion de taille qui, lors de l'utilisation, entoure la taille d'un utilisateur et une portion d'entrejambe reliant les portions avant et arrière de la portion de taille, la portion d'entrejambe comprenant une âme absorbante pour absorber des exsudats corporels et une feuille supérieure perméable aux liquides recouvrant l'âme absorbante tout en indiquant une zone d'absorption où l'âme absorbante est située, où l'article a une surface intérieure qui, lors de l'utilisation, fait face à la peau de l'utilisateur et une surface extérieure qui est opposée à la surface intérieure et, lors de l'utilisation, fait face aux vêtements de l'utilisateur; où l'article est composé d'une pluralité de tissus qui définissent les couleurs des surfaces intérieures et extérieures de l'article, la feuille supérieure perméable aux liquides étant l'un des tissus ; où une zone d'absorption est définie comme la partie de la surface intérieure, sur la feuille supérieure, sous laquelle l'âme absorbante est située, et où une zone environnante est définie comme la partie de la surface intérieure, sur la feuille supérieure, adjacente à et au moins entourant en partie la zone d'absorption ; où

pour chaque premier article de culotte jetable, au moins la surface extérieure et ladite zone environnante ont sensiblement la même première couleur avec, dans l'espace de couleur CIE L*a*b*, des valeurs L* inférieures à 80 et des différences de couleur mutuelles ΔE*ab inférieures à 10 ;

pour chaque deuxième article de culotte jetable, au moins la surface extérieure et ladite zone environnante ont sensiblement la même deuxième couleur avec des valeurs L* inférieures à 80 et des différences de couleurs mutuelles ΔE*ab inférieures à 10 ;

ladite surface extérieure dudit premier et/ou deuxième article de culotte jetable comprend des portions de surface extérieure, formant ensemble ladite surface extérieure, et ladite surface intérieure dudit premier et/ou deuxième article de culotte jetable comprend des portions de surface intérieure, formant ensemble ladite surface intérieure, et toutes les portions de surface extérieure et intérieure dudit premier article, éventuellement sauf dans ladite zone d'absorption, ont des valeurs L* inférieures à 80 et des différences de couleurs mutuelles ΔE*ab inférieures à 10 et/ou toutes les portions de surface extérieure et intérieure dudit deuxième article, éventuellement sauf dans ladite zone d'absorption, ont des valeurs L* inférieures à 80 et des différences de couleurs mutuelles ΔE*ab inférieures à 10 ;

chacun des premier et deuxième articles de culotte jetable comprend une étiquette d'information disposée à l'intérieur de ladite portion de taille et fournissant un indicateur visuel pour différencier entre les portions avant et arrière de l'article respectif, le matériau de l'étiquette d'information étant d'une troisième couleur qui est différente des première et deuxième couleurs par une différence de couleur ΔE*ab supérieure à 20 ; et

la première structure est structurellement différente de la deuxième structure et/ou la première couleur est différente de la deuxième couleur par une différence de couleur ΔE*ab supérieure à 10.

2. Réseau selon la revendication 1, où toutes les portions de surface intérieure et extérieure dudit premier article, sauf dans ladite zone d'absorption, ont des valeurs L* inférieures à 80 et des différences de couleur mutuelles ΔE*ab inférieures à 10 et/ou toutes les portions de surface intérieure et extérieure dudit deuxième article, sauf dans ladite zone d'absorption, ont des valeurs L* inférieures à 80 et des différences de couleur mutuelles ΔE*ab inférieures à 10.

3. Réseau selon la revendication 1, où toutes les portions de surface intérieure et extérieure dudit premier article, sauf dans ladite zone d'absorption, ont des valeurs L* inférieures à 60 et des différences de couleurs mutuelles ΔE*ab inférieures à 10 et/ou toutes les portions de surface intérieure et extérieure dudit deuxième article, sauf dans ladite zone d'absorption, ont des valeurs L* inférieures à 60 et des différences de couleur mutuelles ΔE*ab inférieures à 10.

4. Réseau selon la revendication 1, où toutes les portions de surface intérieure et extérieure dudit premier article ont des valeurs L* inférieures à 80 et des différences de couleurs mutuelles ΔE*ab inférieures à 10 et/ou toutes les portions de surface intérieure et extérieure du deuxième article de culotte jetable ont des valeurs L* inférieures à 80 et des différences de couleur mutuelles ΔE*ab inférieures à 10.

5. Réseau selon la revendication 1, où toutes les portions de surface intérieure et extérieure dudit premier article ont des valeurs L* inférieures à 60 et des différences de couleurs mutuelles ΔE*ab inférieures à 10 et/ou toutes les portions de surface intérieure et extérieure du deuxième article de culotte jetable ont des valeurs L* inférieures à 60 et des différences de couleur mutuelles ΔE*ab inférieures à 10.

6. Réseau selon la revendication 1, où la zone d'absorption dudit premier et/ou deuxième article de culotte jetable a une valeur L* inférieure à 90 et une différence de couleur ΔE*ab supérieure à 5 par rapport à ladite zone environnante et/ou à ladite surface extérieure.

7. Réseau selon l'une quelconque des revendications 1 à 6, où la différence structurelle est choisie parmi: le premier article est optimisé pour les utilisateurs masculins tandis que le deuxième article est optimisé pour les utilisateurs féminins; les premier et deuxième articles ont des tailles différentes; le premier article est un article de culotte fileté comprenant des fils élastiques dans la portion de taille, tandis que le deuxième article n'est pas un tel article de culotte fileté ; le premier article est un article de culotte à couches comprenant un stratifié élastique dans la portion de taille, tandis que le deuxième article n'est pas un tel article de culotte à couches.

8. Réseau selon l'une quelconque des revendications précédentes, où les première et deuxième couleurs sont sensiblement les mêmes.

9. Réseau selon l'une quelconque des revendications 1 à 7, où les première et deuxième couleurs sont différentes d'une différence de couleur ΔE*ab supérieure à 10.

10. Réseau selon l'une quelconque des revendications précédentes, où les première et deuxième couleurs ont des valeurs L* inférieures à 60.

11. Réseau selon l'une quelconque des revendications précédentes, où pour chacun desdits premier et deuxième articles, l'étiquette d'information est disposée sur la portion arrière de la portion de taille.

12. Réseau selon l'une quelconque des revendications précédentes, où pour chacun desdits premier et deuxième articles, l'âme absorbante est visible à travers la feuille supérieure perméable aux liquides.

13. Réseau selon l'une quelconque des revendications précédentes, où lesdites étiquettes d'information sont imprimées ou en relief.

14. Réseau selon l'une quelconque des revendications précédentes, où pour chacun desdits premier et deuxième articles, des codes de lot ou des numéros de lot ou des codes de production sont situés sur lesdites étiquettes d'information.

15. Procédé de production d'un réseau d'articles de culotte jetable, comprenant les étapes de :

produire une pluralité de premiers articles ayant une première structure, comprenant une portion de taille qui, lors de l'utilisation, entoure la taille d'un utilisateur et une portion d'entrejambe reliant les portions avant et arrière de la portion de taille, la portion d'entrejambe comprenant une âme absorbante pour absorber des exsudats corporels et une feuille supérieure perméable aux liquides recouvrant l'âme absorbante, où l'article a une surface intérieure qui, lors de l'utilisation, fait face à la peau de l'utilisateur et une surface extérieure qui est opposée à la surface intérieure et, lors de l'utilisation, fait face aux vêtements de l'utilisateur ; où l'article est composé d'une pluralité de tissus qui définissent les couleurs des surfaces intérieure et extérieure de l'article, la feuille supérieure perméable aux liquides étant l'un des tissus ; où une zone d'absorption est définie comme la partie de la surface intérieure, sur la feuille supérieure, sous laquelle est située l'âme absorbante, et où une zone environnante est définie comme la partie de la surface intérieure, sur la feuille supérieure, adjacente à et au moins entourant en partie la zone d'absorption; où pour chaque premier article de culotte jetable, au moins la surface extérieure et ladite zone environnante ont sensiblement la même première couleur avec, dans l'espace de couleur CIE L*a*b*, des valeurs L* inférieures à 80 et des différences de couleur mutuelles ΔE*ab inférieures à 10 ;
produire une pluralité de deuxièmes articles ayant une première structure, comprenant une portion de taille qui, lors de l'utilisation, entoure la taille d'un utilisateur et une portion d'entrejambe reliant les portions avant et arrière de la portion de taille, la portion d'entrejambe comprenant une âme absorbante pour absorber des exsudats corporels et une feuille supérieure perméable aux liquides recouvrant l'âme absorbante, où l'article a une surface intérieure qui, lors de l'utilisation, fait face à la peau de l'utilisateur et une surface extérieure qui est opposée à la surface intérieure et, lors de l'utilisation, fait face aux vêtements de l'utilisateur ; où l'article est composé d'une pluralité de tissus qui définissent les couleurs des surfaces intérieure et extérieure de l'article, la feuille supérieure perméable aux liquides étant l'un des tissus ; où une zone d'absorption est définie comme la partie de la surface intérieure, sur la feuille supérieure, sous laquelle est située l'âme absorbante, et où une zone environnante est définie comme la partie de la surface intérieure, sur la feuille supérieure, adjacente à et au moins entourant en partie la zone d'absorption; où pour chaque deuxième article de culotte jetable, au moins la surface extérieure et ladite zone environnante ont sensiblement la même deuxième couleur avec des valeurs L* inférieures à 80 et des différences de couleurs mutuelles ΔE*ab inférieures à 10 ;
où ladite surface extérieure dudit premier et/ou deuxième article de culotte jetable comprend des portions de surface extérieure, formant ensemble ladite surface extérieure, et ladite surface intérieure dudit premier et/ou deuxième article de culotte jetable comprend des portions de surface intérieure, formant ensemble ladite surface intérieure, et toutes les portions de surface extérieure et intérieure dudit premier article, éventuellement sauf dans ladite zone d'absorption, ont des valeurs L* inférieures à 80 et des différences de couleurs mutuelles ΔE*ab inférieures à 10 et/ou toutes les portions de surface extérieure et intérieure dudit deuxième article, éventuellement, sauf dans ladite zone d'absorption, ont des valeurs L* inférieures à 80 et des différences de couleurs mutuelles ΔE*ab inférieures à 10 ;
où pour chacun desdits premier et deuxième articles de culotte jetable, la première structure est structurellement différente de la deuxième structure et/ou la première couleur est différente de la deuxième couleur par une différence de couleur ΔE*ab supérieure à 10 ; et

attacher des étiquettes d'informations à l'intérieur desdites portions de taille de chacun des premier et deuxième articles, fournissant ainsi un indicateur visuel pour différencier entre les portions avant et arrière de l'article respectif ; le matériau de l'étiquette d'information étant dans une troisième couleur qui est différente des première et deuxième couleurs par une différence de couleur $\Delta E^*ab$ supérieure à 20.

# Fig. 1

Fig. 2

# Fig. 3

# Fig. 4

# Fig. 5

# Fig. 6

# Fig. 7

Fig. 8B

Fig. 8A

# Fig. 9

Fig. 10

# Fig. 11A  Fig. 11B  Fig. 11C

# Fig. 11D  Fig. 11E  Fig. 11F

# Fig. 12

## Fig. 13A

101

131a

104

131b

131c

131

131d

131e

## Fig. 13B

101'

131'a

104

131'b

131'c

131

131'd

131'e

131'g   131'f

Fig. 14A

Fig. 14B

Fig. 15A

Fig. 15B

Fig. 16A

Fig. 16B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010050854 A1
- WO 2010050853 A1
- WO 2016182484 A1
- EP 1704842 A1
- WO 2007024327 A1
- US 2012323204 A1
- WO 2006015207 A2

- WO 2018097770 A1
- WO 2005122985 A
- WO 2007133127 A
- WO 2016068764 A
- WO 20030028166 A
- US 20090275911 A
- WO 03047488 A